# EUROPEAN PATENT APPLICATION

(11) **EP 4 813 339 A1**
(43) Date of publication of application: **30.09.2026**
(21) Application number: 26167913.8
(22) Date of filing: 26.03.2026
(51) Int. Cl.: A61B 34/35, B25J 9/00, G05B 19/427, A61B 34/00, A61B 34/20, A61B 34/37, A61B 90/00

(54) **SYSTEM AND CONTROL METHOD**

(30) Priority: 27.03.2025 JP 2025054531
(71) Applicant: Sysmex Corporation, Kobe-shi, Hyogo 651-0073 (JP); Keio University, Tokyo, 108-8345 (JP)
(72) Inventor: OHASHI, Masanao, 651-0073, Hyogo (JP); MATSUDA, Satoru, Tokyo, 160-8582 (JP); KITAGAWA, Yuko, Tokyo, 160-8582 (JP)
(74) Representative: Becker, Eberhard

(57) **Abstract**

Disclosed is a system for a surgical assistance robot configured to perform a surgical procedure on a patient using a surgical instrument attached to an arm, the system comprising:
a console comprising (i) a hand controller configured to be manipulated by a surgeon to remotely control an operation of the surgical instrument, wherein the hand controller comprises a plurality of joints rotatable by a manipulation of the hand controller, and (ii) a viewer configured to display images captured by an endoscope, the images showing a surgical site of the patient and the operation of the surgical instrument;
a storage configured to store (a) a video generated from the images which are captured during the surgical procedure and (b) a manipulation log representing a series of the manipulations of the hand controller during the surgical procedure, wherein the manipulation log comprises an angle of rotation of each of the joints according to the manipulation; and
a transmitter configured to transmit, to the console, the video and the manipulation log stored in the storage,
wherein the console is configured to rotate the joints according to the manipulation log in association with a playback of the video on the viewer for duplication of the manipulations by the surgeon during the surgical procedure.

## Description

### TECHNICAL FIELD

The present invention relates to a system and a control method for a surgical assistance robot.

### BACKGROUND

Conventionally, surgical procedures using a surgical assistance robot have been performed. A surgical procedure using a surgical assistance robot is performed by an operator manipulating a console to operate an arm having a surgical instrument attached to a distal end thereof. To ensure the reliability of surgical procedures for patients, it is desired to improve the operator's proficiency in manipulating the console.

U.S. Patent No. 11315438 discloses a system for training in surgical procedures using a surgical assistance robot. This system includes an operation console used by a surgeon who performs a surgical procedure and a training console used by a surgeon who receives training, and while the surgical procedure is being performed via the operation console, a video showing a surgical site is displayed on a display of the training console. The training console, for example, divides the display screen of the training console into two and displays a video of the surgical procedure currently being performed and a simulation screen for practicing each procedure of the surgical procedure side by side.

The conventional training system leaves it difficult to understand how the surgeon was manipulating the console during the surgical procedure simply by viewing a video of the motion of the surgical assistance robot during the surgical procedure.

### SUMMARY

The present disclosure is directed to a system for a surgical assistance robot configured to perform a surgical procedure on a patient using a surgical instrument attached to an arm, the system comprising: a console comprising (i) a hand controller configured to be manipulated by a surgeon to remotely control an operation of the surgical instrument, wherein the hand controller comprises a plurality of joints rotatable by a manipulation of the hand controller, and (ii) a viewer configured to display images captured by an endoscope, the images showing a surgical site of the patient and the operation of the surgical instrument; a storage configured to store (a) a video generated from the images which are captured during the surgical procedure and (b) a manipulation log representing a series of the manipulations of the hand controller during the surgical procedure, wherein the manipulation log comprises an angle of rotation of each of the joints according to the manipulation; and a transmitter configured to transmit, to the console, the video and the manipulation log stored in the storage, wherein the console is configured to rotate the joints according to the manipulation log in association with a playback of the video on the viewer for duplication of the manipulations by the surgeon during the surgical procedure.

The present disclosure is also directed to a method for controlling a console for a surgical assistance robot, the console including a hand controller configured to operate a surgical instrument attached to an arm of the surgical assistance robot and a viewer configured to display images showing a surgical site of a patient and an operation of the surgical instrument, the method comprising: storing (a) a video generated from the images which are captured during a surgical procedure, and (b) a manipulation log representing a series of manipulations by a surgeon of the hand controller during the surgical procedure, wherein the manipulation log comprises an angle of rotation of each of a plurality of joints of the hand controller; and rotating the joints of the hand controller according to the manipulation log in association with a playback of the video on the viewer for duplication of the manipulations by the surgeon during the surgical procedure.

According to the system and method disclosed hereby, the manipulations of the console by the surgeon performed during the surgical procedure can be easily understood.

### BRIEF DESCRIPTION OF THE DRAWINGS

FIG. 1 is a diagram for explaining an overview of a system related to the present invention.
FIG. 2 is a block diagram showing a configuration of a surgical system according to Embodiment 1.
FIG. 3 is a perspective view showing an appearance of an arm apparatus according to Embodiment 1.
FIG. 4 is a block diagram showing a functional configuration of the arm apparatus according to Embodiment 1.
FIG. 5 is a perspective view showing an appearance of a console according to Embodiment 1.
FIG. 6 is a perspective view showing an appearance of a hand controller according to Embodiment 1.
FIG. 7 is a perspective view showing an appearance near a distal end of the hand controller according to Embodiment 1.
FIG. 8 is a perspective view showing an appearance of a foot unit according to Embodiment 1.
FIG. 9 is a perspective view showing a state in which an operator uses the console according to Embodiment 1.
FIG. 10 is a block diagram showing a functional configuration of the console according to Embodiment 1.
FIG. 11 is a block diagram showing a functional configuration of the hand controller according to Embodiment 1.
FIG. 12 is a block diagram showing a functional configuration of a control apparatus according to Embodiment 1.
FIG. 13 is a block diagram showing a functional configuration of an information processing apparatus according to Embodiment 1.
FIG. 14 is a diagram showing the configuration of the surgical system 1 according to Embodiment 1, with information transmitted during a surgical procedure superimposed thereon.
FIG. 15 is a diagram showing an example of manipulation information transmitted from the console to the control apparatus according to Embodiment 1.
FIG. 16 is a diagram showing manipulation information continuously transmitted from the console to the control apparatus at predetermined time intervals according to Embodiment 1.
FIG. 17 is a diagram showing a configuration of a plurality of video files stored in the information processing apparatus according to Embodiment 1.
FIG. 18 is a diagram showing a configuration of a video selection screen displayed on a display of the information processing apparatus according to Embodiment 1.
FIG. 19 is a diagram showing a configuration of an edit screen displayed on the display of the information processing apparatus according to Embodiment 1.
FIG. 20 is a diagram showing display states 1 and 2 of a video control area according to Embodiment 1.
FIG. 21 is a diagram showing a configuration of a playback data processing screen displayed on the display of the information processing apparatus according to Embodiment 1.
FIG. 22 is a diagram showing generation of video data and a manipulation log corresponding to a playback target period according to Embodiment 1.
FIG. 23 is a diagram for explaining details of generation of video data and a manipulation log corresponding to the playback target period according to Embodiment 1.
FIG. 24 is a diagram showing the configuration of the surgical system 1 according to Embodiment 1, with information transmitted from the information processing apparatus to the console superimposed thereon.
FIG. 25 is a diagram showing a configuration of a playback data selection screen displayed on an operation panel of the console according to Embodiment 1.
FIG. 26 is a diagram showing a configuration of an endoscopic video played back and displayed on a viewer of the console according to Embodiment 1.
FIG. 27 is a block diagram showing a configuration of a surgical system according to Embodiment 2.

### DESCRIPTION OF THE EMBODIMENTS

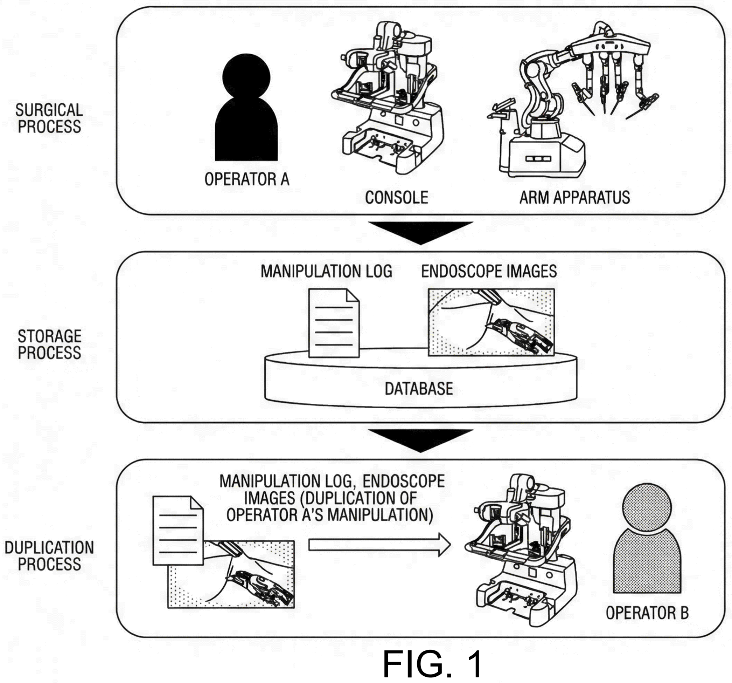

The present invention relates to a system for a surgical assistance robot. A surgical procedure using a surgical assistance robot is performed by a surgeon manipulating a console to operate arms, each having a surgical instrument attached to a distal end thereof. The console includes hand controllers for operating the arms and the surgical instruments attached to the distal ends of the arms. As shown in FIG. 1, in a surgical process for performing a surgical procedure on a patient, when a surgeon (operator A) manipulates hand controllers of a console, surgical instruments attached to distal ends of arms operate to perform the surgical procedure. During the surgical procedure, images captured by an endoscope attached to one of the arms are displayed on a viewer of the console, and the surgeon (operator A) manipulates the console while viewing the endoscopic images. As shown in FIG. 1, in the system according to the present invention, a storing step is performed for storing, in a storage, a manipulation log of the hand controllers and data of endoscopic images (video data) obtained in the surgical process. Since the hand controllers include a plurality of joints, the manipulation log includes information reflecting an angle of rotation of each of the joints according to the manipulations by the surgeon (operator A) during the surgical procedure. In the system according to the present invention, for example, when performing training of a trainee surgeon (operator B), as shown in FIG. 1, the manipulation log and video data stored in the storage are transmitted to a console used by the trainee surgeon (operator B). Then, in order to duplicate the manipulations of the hand controller by the surgeon (operator A) during the surgical procedure, a duplicating step is performed for rotating each joint of the hand controllers according to the manipulation log corresponding to a playback period of the video while playing back the video on the viewer of the console used by the trainee surgeon (operator B).

In the duplicating step, for example, while the trainee surgeon (operator B) sits at the console in a posture for the surgical procedure with his or her fingers lightly touching the hand controllers, each of the joints of the hand controllers is driven according to the manipulation log corresponding to the playback period of the video while playing back the video on the viewer. Accordingly, the trainee surgeon (operator B) sitting at the console can experience the manipulations of the hand controllers actually performed by the surgeon (operator A) during the surgical procedure, while viewing the motion of the surgical instrument at the surgical site of the patient undergoing the surgical procedure through the video. Therefore, even a surgeon who is still unfamiliar with the operation of the console can learn the operation of the console at that time while viewing, for example, a video of a surgical procedure performed by a highly proficient expert surgeon, and can be expected to enhance his or her own operation skills. In addition, the system according to the present invention can not only perform confirmation of surgical procedures performed by other surgeons but also, for example, by playing back video and manipulation logs of the surgeon's own past surgical procedure, allow the surgeon to look back on how the console was operated in the past surgical procedure and enhance operation skills.

The system according to the present invention will be described in detail below.

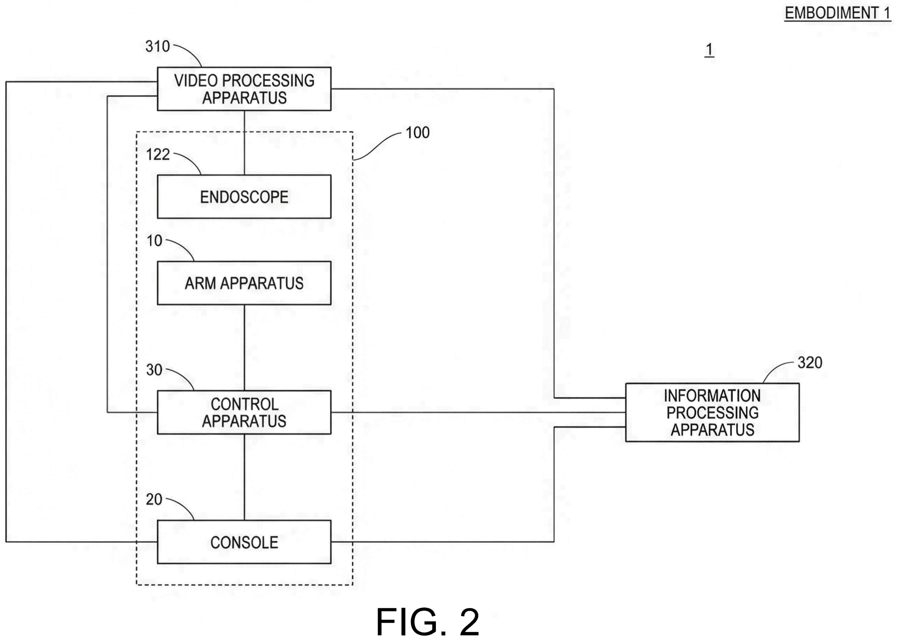

<Embodiment 1> FIG. 2 is a block diagram showing a configuration of a surgical system 1 according to Embodiment 1 of the present invention.

The surgical system 1 includes a surgical assistance robot 100, a video processing apparatus 310, and an information processing apparatus 320.

The surgical assistance robot 100 is an apparatus used for endoscopic surgery, for example, minimally invasive surgery such as laparoscopic surgery. The surgical assistance robot 100 includes an arm apparatus 10 including arms, each having a surgical instrument attached at a distal end thereof, a console 20 manipulated to operate the arm apparatus 10, and a control apparatus 30. The control apparatus 30 controls the arm apparatus 10 according to manipulations of the console 20. An endoscope 122 is detachably attached to the arm apparatus 10.

The console 20 and the control apparatus 30 are communicatively connected to the video processing apparatus 310. The video processing apparatus 310 generates video data from an image obtained by the endoscope 122 during a surgical procedure using the surgical assistance robot 100, and transmits the generated video data to the console 20. Further, the video processing apparatus 310 is communicatively connected to the information processing apparatus 320, and transmits the generated video data to the information processing apparatus 320. The control apparatus 30 is also communicatively connected to the information processing apparatus 320 in order to transmit the manipulation log of the console 20 to the information processing apparatus 320.

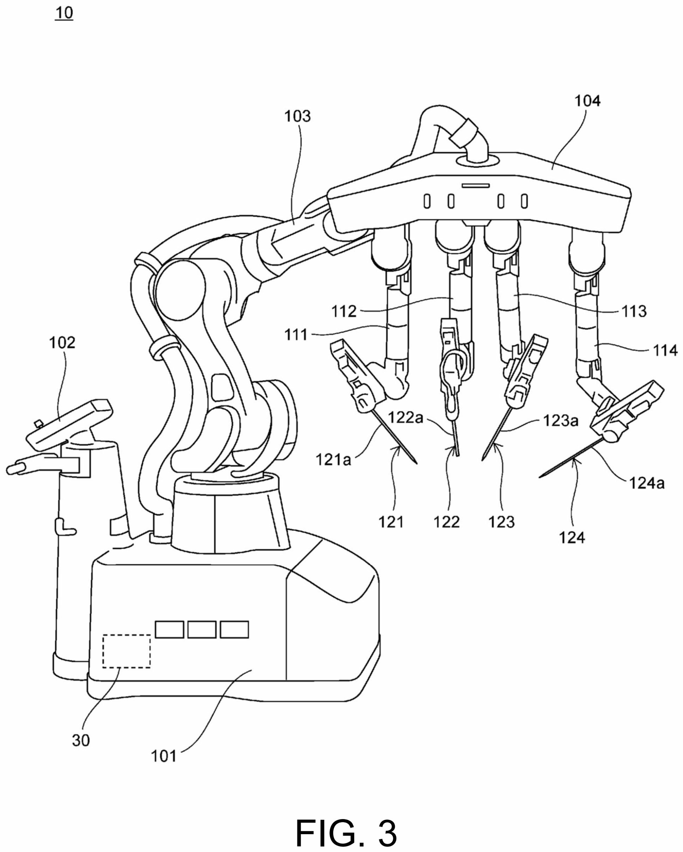

FIG. 3 is a perspective view showing an appearance of the arm apparatus 10.

The arm apparatus 10 includes a plurality of arms, each of which can have a surgical instrument such as forceps or an endoscope. The arm apparatus 10 is disposed near a patient. The arm apparatus 10 operates according to driving instructions transmitted from the control apparatus 30 in response to manipulating the console 20 by an operator (for example, a surgeon).

The arm apparatus 10 includes a base 101, an operation unit 102, a base arm 103, a support unit 104, and arms 111 to 114. Surgical instruments 121, 123, and 124 are detachably attached to distal ends of the arms 111, 113, and 114, respectively, and an endoscope 122 is detachably attached to a distal end of the arm 112.

The base 101 has the control apparatus 30 built therein. The operation unit 102 is installed on the base 101 and includes a display and an input unit. An operator of the arm apparatus 10 operates the input unit of the operation unit 102 to adjust moving speeds and the like of the arms 111 to 114 of the arm apparatus 10 while viewing the display of the operation unit 102. The base arm 103 is an arm installed on the base 101 at its rear end. The base arm 103 includes a plurality of joints. The support unit 104 is rotatably connected to a distal end of the base arm 103. The support unit 104 moves along with the movement of the distal end of the base arm 103 and rotates about an axis of the distal end of the base arm 103.

The arms 111 to 114 have upper ends installed on a lower surface of the support unit 104. Each of the arms 111 to 114 is an arm provided with a plurality of axes (e.g., twelve axes) and a plurality of joints corresponding to the twelve axes. Holders each configured to detachably mount a surgical instrument or an endoscope are provided at lower ends of the arms 111 to 114, and the surgical instrument or the endoscope is attached to the holders.

Each of the surgical instruments and the endoscope is provided with elongated shaft portions 121a to 124a, and during a surgical procedure using the surgical assistance robot 100, the shaft portions 121a to 124a are inserted into a body of a patient via a guide tube (trocar) inserted into an abdomen of the patient. In the present embodiment, the surgical instruments 121, 123, and 124 are forceps. For example, the surgical instruments (forceps) 121 and 124 are so-called grasping forceps. The surgical instrument (forceps) 123 is a so-called electric scalpel, and includes a hand portion capable of incision and coagulation at a distal end thereof. The endoscope 122 is, for example, a 3D videoscope.

The forceps is not limited to grasping forceps, and may be other types of forceps such as hemostatic forceps or dissecting and ligating forceps, or may be an electric instrument such as an electric scalpel. Each surgical instrument may be replaced with a different type of surgical instrument as needed during the surgical procedure.

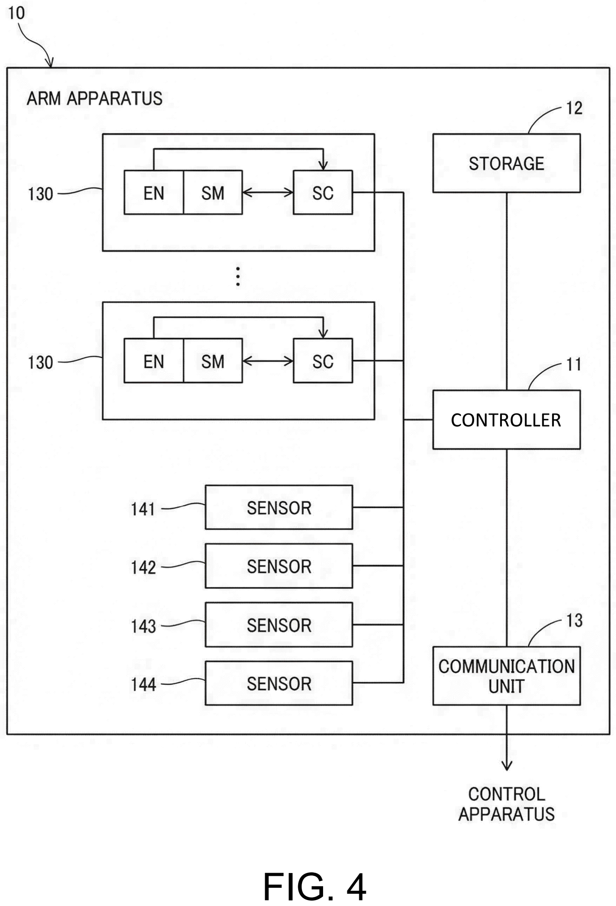

FIG. 4 is a block diagram showing a functional configuration of the arm apparatus 10.

The arm apparatus 10 includes a controller 11, a storage 12, a communication unit 13, a plurality of actuators 130, and sensors 141 to 144.

The controller 11 includes, for example, a CPU or an FPGA. The controller 11 controls each part of the hardware of the arm apparatus 10 and transmits and receives signals to and from the control apparatus 30. The storage 12 is composed of, for example, an HDD or an SSD. The communication unit 13 includes a communication interface capable of communicating with the control apparatus 30 based on a predetermined communication standard.

One actuator 130 corresponds to one of a plurality of joints included in the arms 111 to 114. Each actuator 130 includes a servo motor SM, an encoder EN, and a servo controller SC. The controller 11 controls the servo controller SC of the corresponding actuator 130 based on driving instructions received from the control apparatus 30 via the communication unit 13. In response to the control of the controller 11, the servo controller SC drives the servo motor SM so that an output signal of the encoder EN reaches a desired value. Sensors 141 to 144 are installed in the arms 111 to 114, respectively. Sensors 141 to 144 detect attachment and detachment of a surgical instrument or an endoscope to and from the arms 111 to 114.

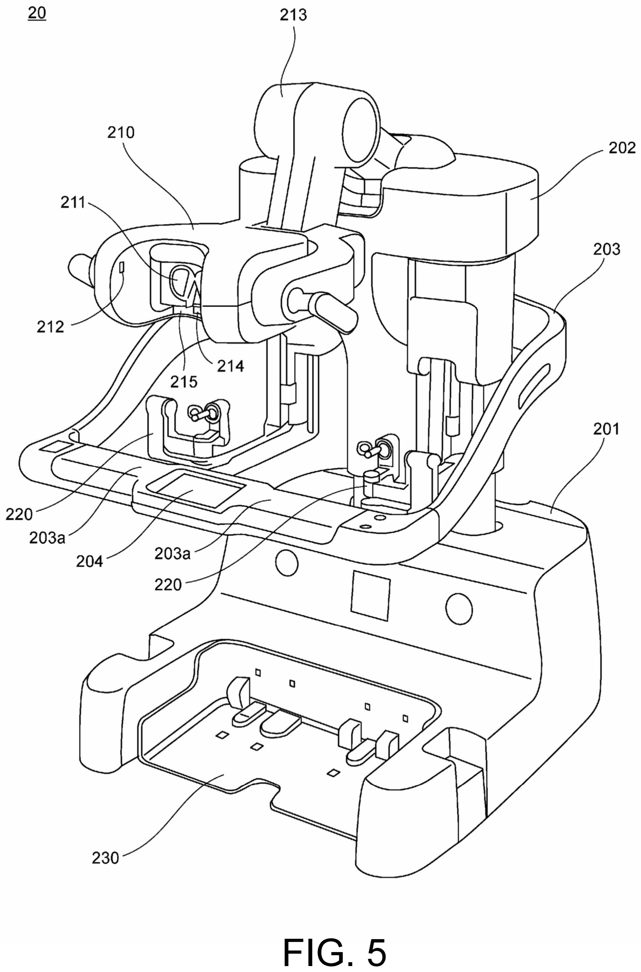

FIG. 5 is a perspective view showing an appearance of the console 20.

The console 20 is an apparatus disposed near an operator (for example, a surgeon) such as a doctor. The console 20 is an apparatus for driving the arm apparatus 10 in response to being manipulated by the operator during a surgical procedure using the surgical assistance robot 100.

The console 20 includes a base 201, a support unit 202, a frame member 203, an operation panel 204, a viewer unit 210, two hand controllers 220, and a foot unit 230.

The support unit 202 is installed on an upper part of the base 201 so as to be movable up and down. The frame member 203 is installed on the support unit 202. The operation panel 204 is installed on an upper surface of a front center of the frame member 203. The operation panel 204 is a display input unit that performs display of images and acceptance of operations, and is, for example, a touch panel display. To left and right of the operation panel 204, armrests 203a are formed to allow the operator to rest his or her elbows when manipulating the hand controllers 220. The height of the armrest 203a can be changed by moving the support unit 202 up and down relative to the base 201.

The viewer unit 210 is supported by a distal end of an arm 213 installed on the upper surface of the support unit 202. The height of the viewer unit 210 is adjustable through rotation of a joint of the arm 213. The viewer unit 210 includes a viewer 211, head sensors 212, the arm 213, a microphone 214, and a speaker 215.

The viewer 211 displays images captured by the endoscope 122. The head sensors 212 are provided so as to sandwich an area in front of the viewer 211. The head sensors 212 are a transmission-type photoelectric sensor. When the operator looks into the viewer 211, the head of the operator approaches the viewer 211, light between the head sensors 212 is blocked, and a state in which the operator is viewing the viewer 211 is detected. The microphone 214 converts audio uttered by the operator into audio data. The speaker 215 outputs audio based on the audio data. The operator can use the microphone 214 and the speaker 215 to have a conversation with a surgical assistant located near the arm apparatus 10.

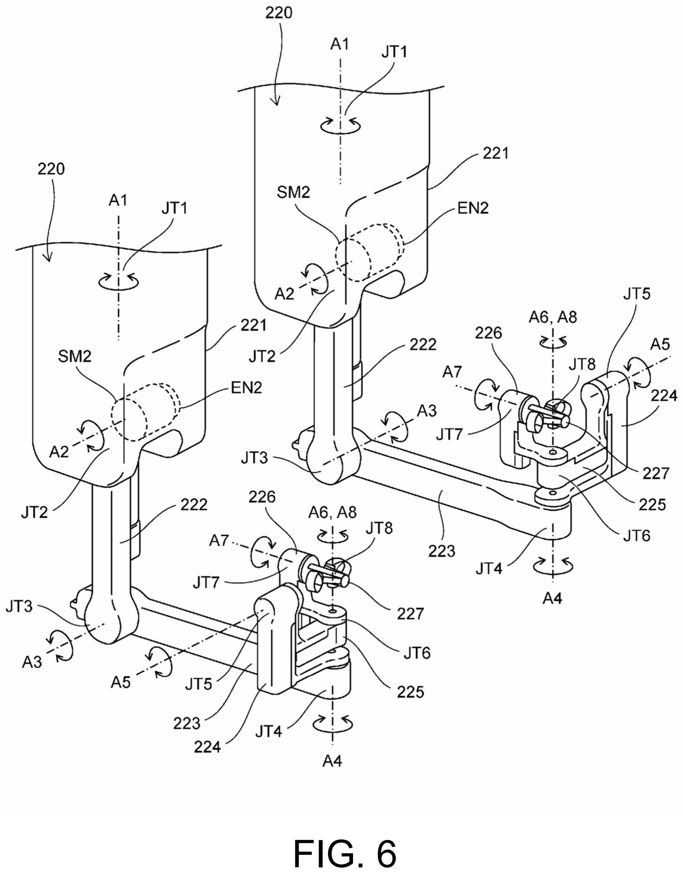

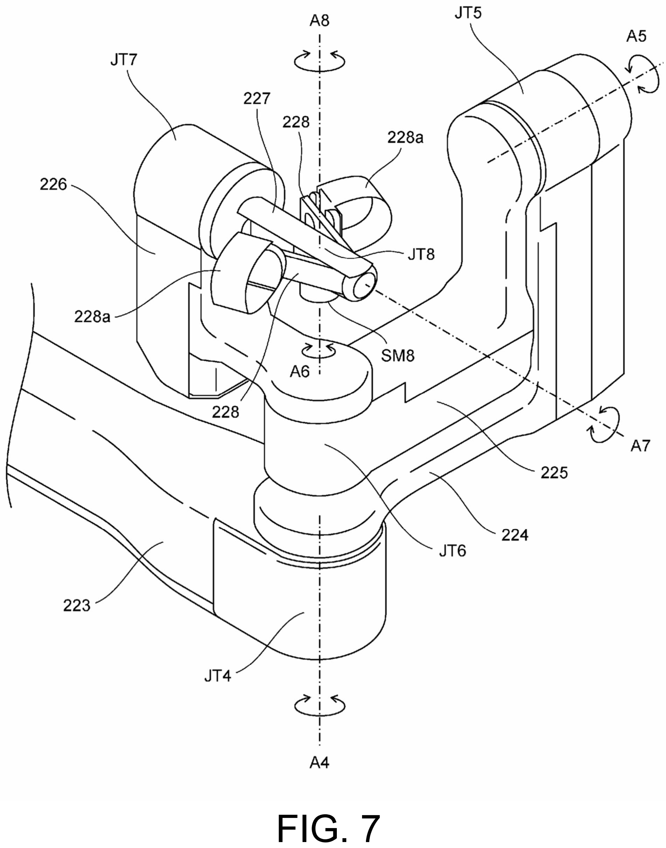

The hand controllers 220 for the left and right hands are installed on the support unit 202 and are input devices for the operator to manipulate the arms 111 to 114 of the arm apparatus 10 using the left hand and the right hand, respectively. Each hand controller 220 comprises eight axes and joints corresponding to the eight axes. A configuration of the hand controller 220 will be described later with reference to FIGS. 6 and 7.

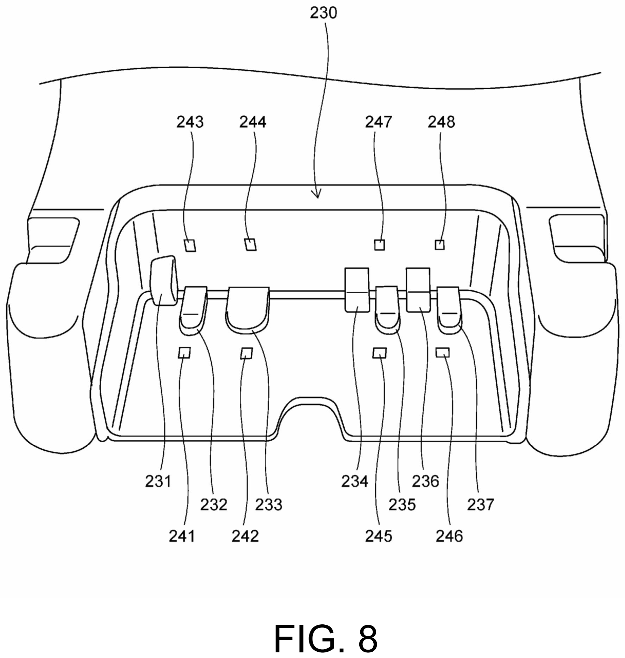

The foot unit 230 is installed so as to be movable back and forth at a lower front portion of the base 201, and is an input device for the operator to manipulate the arm apparatus 10 using a left foot and a right foot. A configuration of the foot unit 230 will be described later with reference to FIG. 8.

FIG. 6 is a perspective view showing an appearance of the hand controller 220. FIG. 7 is a perspective view showing an appearance near a distal end of the hand controller 220.

As shown in FIG. 6, the two hand controllers 220 are configured symmetrically in a left-right direction. Hereinafter, the configuration of one of the hand controllers 220 will be described with reference to FIGS. 6 and 7.

The hand controller 220 includes link portions 221, 222, 223, 224, 225, 226, a support shaft 227, a pair of movable plates (grips) 228, and joints JT1, JT2, JT3, JT4, JT5, JT6, JT7, JT8. The link portions 224 to 226 have an L-shape.

The link portion 221 is installed on the support unit 202 (see FIG. 5) so as to be rotatable about an axis A1 along a vertical direction. An upper end of the link portion 222 is installed on a lower end of the link portion 221 so as to be rotatable about an axis A2 along a horizontal direction. One end of the link portion 223 is installed on the lower end of the link portion 222 so as to be rotatable about an axis A3 along the horizontal direction. One end of the link portion 224 is installed on the other end of the link portion 223 so as to be rotatable about an axis A4. One end of the link portion 225 is installed on the other end of the link portion 224 so as to be rotatable about an axis A5. One end of the link portion 226 is installed on the other end of the link portion 225 so as to be rotatable about an axis A6. One end of the support shaft 227 is installed on the other end of the link portion 226 so as to be rotatable about an axis A7. As shown in FIG. 7, a pair of movable plates 228 is installed on the other end of the support shaft 227 so as to be rotatable about an axis A8. The axes A6 and A8 coincide with each other.

The link portion 221 is connected to the support unit 202 (see FIG. 5) by a joint JT1. The link portions 221 and 222 are connected by a joint JT2. The link portions 222 and 223 are connected by a joint JT3. The link portions 223 and 224 are connected by a joint JT4. The link portions 224 and 225 are connected by a joint JT5. The link portions 225 and 226 are connected by a joint JT6. The link portion 226 and the support shaft 227 are connected by a joint JT7. As shown in FIG. 7, the support shaft 227 and the pair of movable plates 228 are connected by a joint JT8. The joints JT1 to JT8 rotate about axes A1 to A8, respectively.

As shown in FIG. 7, the pair of movable plates 228 have an elongated plate shape and is installed so as to flank the support shaft 227. The pair of movable plates 228 rotates about the axis A8. The movable plate 228 includes a cylindrical finger insertion portion 228a. The finger insertion portion 228a is implemented by, for example, a hook-and-loop fastener. The pair of movable plates 228 is a portion directly manipulated by an operator using fingers, and is an operation target with which the operator comes into contact for manipulation. The operator manipulates the hand controller 220 by inserting fingers into the pair of finger insertion portions 228a. A spring is installed between the pair of movable plates 228, and as shown in FIG. 7, an opening angle between the pair of movable plates 228 becomes a predetermined angle in a state where there is no load.

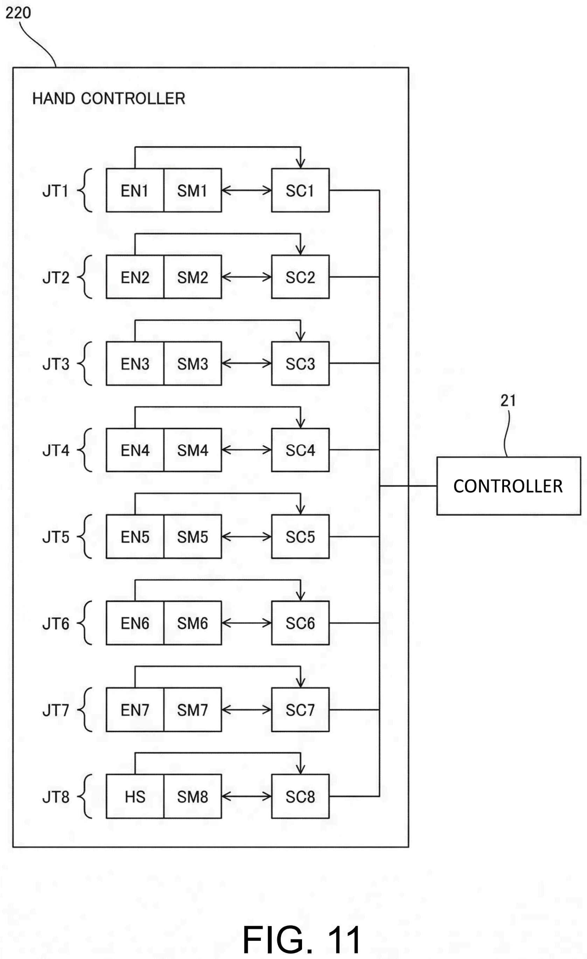

The hand controller 220 further includes servo motors SM1 to SM8 (see FIG. 11) respectively corresponding to the joints JT1 to JT8, encoders EN1 to EN7 (see FIG. 11) respectively corresponding to the joints JT1 to JT7, and a Hall sensor HS (see FIG. 11) corresponding to the joint JT8. For convenience, FIG. 6 shows a servo motor SM2 and an encoder EN2 corresponding to the joint JT2, and FIG. 7 shows a servo motor SM8 corresponding to the joint JT8.

The servo motors SM1 to SM8 rotate the joints JT1 to JT8 about corresponding axes, respectively. The encoders EN1 to EN7 output signals corresponding to rotation angles of the joints JT1 to JT7 about corresponding axes, respectively. The Hall sensor HS is installed on the support shaft 227, and a magnet is installed on one of the movable plates 228. When the operator opens and closes the pair of movable plates 228, the magnet and the Hall sensor HS function as an angle detection sensor, and the Hall sensor HS outputs a signal corresponding to an opening angle of the pair of movable plates 228.

For detecting the opening angle of the pair of movable plates 228, various detectors such as an encoder and a sensor that output a signal corresponding to a state such as a position, a rotation amount, an angle, and the like of the pair of movable plates 228 may optionally be used instead of the Hall sensor HS.

Before starting a surgical procedure, an operator such as a doctor fixes fingers of a left hand and fingers of a right hand to the controllers 220 for the left and right hand, respectively. That is, the thumbs and the index fingers or middle fingers are pressed against the pair of movable plates 228 so as to sandwich the pair of movable plates 228. In this state, the finger insertion portions 228a are fastened to the fingers sandwiching the pair of movable plates 228.

During the surgical procedure, when the operator performs a manipulation to move the support shaft 227, the joints JT1 to JT7 of the hand controller 220 rotate about their axes according to the movement of the support shaft 227. Then, based on output values of the encoders EN1 to EN7, target arms of the arm apparatus 10 are driven, and a surgical instrument attached to each of the arms operate. Further, in a case where forceps are attached to the target arms, when the operator performs a manipulation to open and close the pair of movable plates 228, a distal end portion of the forceps attached to the arms are driven to open and close based on an output value of the Hall sensor HS.

FIG. 8 is a perspective view showing an appearance of the foot unit 230.

The foot unit 230 includes foot pedals 231 to 237 and foot sensors 241 to 248.

An operator performs an input for operating the arm apparatus 10 by stepping on the foot pedals 231 to 237 with a foot. That is, the foot pedals 231 to 237 are operating elements for performing predetermined inputs. The foot pedals 234 to 237 are operating elements for switching between energization and de-energization of an electric scalpel when the electric scalpel is attached to a target arm.

When the foot pedal 231 is stepped on, a surgical instrument to be operated by the right hand controller 220 is switched between the surgical instrument 123 and the surgical instrument 124. The foot pedal 232 is a clutch pedal. While the foot pedal 232 is being stepped on, transmission of movement of the hand controller 220 to the arm apparatus 10 is interrupted. The foot pedal 233 is a camera pedal. While the foot pedal 233 is being stepped on, manipulation of an arm equipped with the endoscope 122 is enabled by both hand controllers 220.

The foot pedals 234 and 236 are cutting pedals. While the foot pedals 234 and 236 are being stepped on, a high frequency is applied to a distal end of the forceps (electric scalpel) so as to enable cutting by the forceps (electric scalpel). The foot pedals 235 and 237 are coagulation pedals. While the foot pedals 235 and 237 are being stepped on, a high frequency is applied to the distal end of the forceps (electric scalpel) so as to enable coagulation by the forceps (electric scalpel).

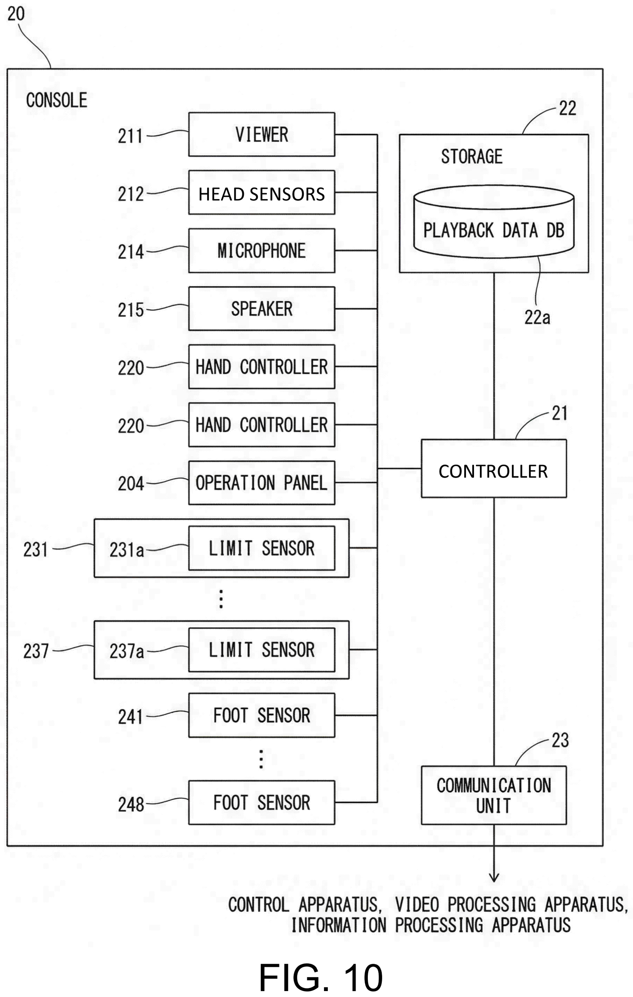

In addition, each of the foot pedals 231 to 237 is provided with a limit sensors 231a to 237a (see FIG. 10) for detecting whether or not the corresponding foot pedal has been stepped on. Thereby, it is detected whether or not the foot pedals 231 to 237 are being stepped on.

The foot sensors 241 to 248 are reflective photoelectric sensors for detecting a position of a foot of the operator. Whether or not a left foot is positioned at the foot pedals 231 to 233 is detected by the foot sensors 241 to 244, and whether or not a right foot is positioned at the foot pedals 234 to 237 is detected by the foot sensors 245 to 248. Specifically, the foot sensors 241 to 248 detect (i) a hover state, including a state in which a foot is in front of a foot pedal, a state in which a foot is above a foot pedal, and a state in which a foot is stepping on a foot pedal, and (ii) a non-hover state in which a foot is not inside the foot unit 230.

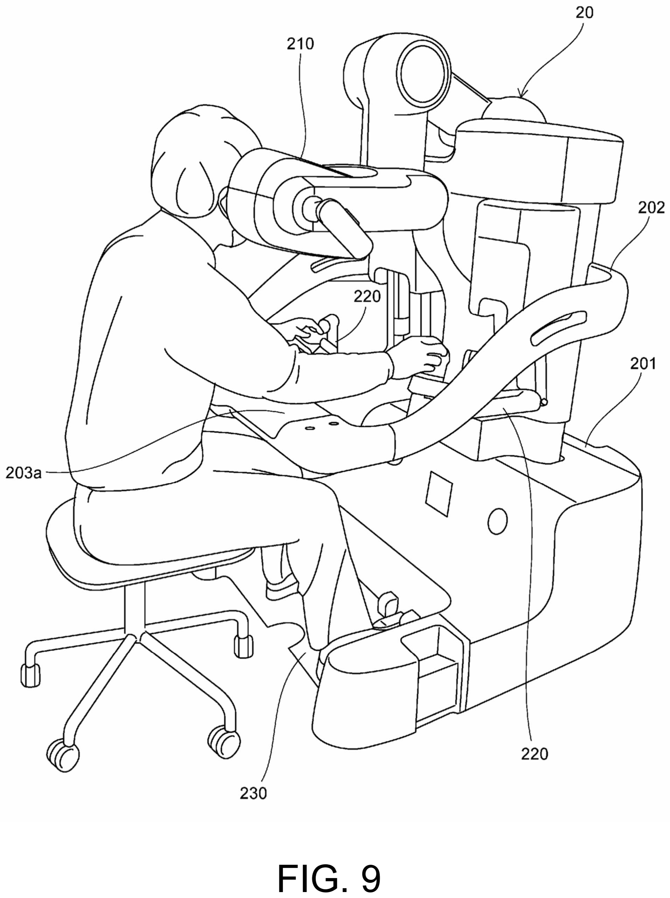

FIG. 9 is a perspective view showing a state in which an operator uses the console 20.

Before starting the surgical procedure, the patient lying on an operating table is positioned below surgical instruments attached to the arm apparatus 10, a guide tube (trocar) is inserted into an abdomen of the patient, and the surgical instruments are inserted into a body of the patient via the guide tube (trocar). While sitting in a chair, the operator places both arms on the armrests 203a and places both feet inside or in front of the foot unit 230. The operator fixes left and right fingers to the controllers 220 for the left and right hand, respectively. Then, the operator places his/her head into the viewer unit 210 and looks into the viewer 211. In response to detecting by the head sensors 212 (see FIG. 5) that the head of the operator is positioned within the viewer unit 210, manipulation of the arm apparatus 10 by the console 20 is enabled.

When the surgical procedure is started, the operator manipulates the left and right support shafts 227 and the pair of movable plates 228 (see FIG. 7) to input motion instructions for operating tips of the forceps. The motion instructions include an instruction for a grasping motion of grasping an object with the tips of the forceps. According to the motion instructions input to the console 20 by the operator, the arms 111 to 114 of the arm apparatus 10 and the surgical instruments and endoscope attached to the arms 111 to 114 are driven. In this way, various surgical procedures are performed.

The operator removes his/her head from the viewer unit 210 when taking a break, ending the surgical procedure, or the like. When it is detected by the head sensors 212 that the head of the operator is not positioned within the viewer unit 210, manipulation of the arm apparatus 10 by the console 20 is disabled. This prevents a malfunction of the arm apparatus 10. Voice uttered by the operator during the surgical procedure is converted into audio data by the microphone 214 (see FIG. 5) and output from a speaker near the arm apparatus 10. Thereby, the operator can give instructions to a surgical assistant located near the arm apparatus 10.

FIG. 10 is a block diagram showing a functional configuration of the console 20.

The console 20 includes a controller 21, a storage 22, a communication unit 23, a viewer 211, a head sensor 212, a microphone 214, a speaker 215, left and right hand controllers 220, an operation panel 204, foot pedals 231 to 237, and foot sensors 241 to 248.

The controller 21 is composed of, for example, a CPU or an FPGA. The controller 21 controls each part of the hardware of the console 20 and transmits and receives signals to and from the video processing apparatus 310 and the control apparatus 30. The storage 22 is composed of, for example, an HDD or an SSD. A playback data DB (playback data database) 22a is stored in the storage 22. Video data and a manipulation log for playback on the console 20 are stored in the playback data DB 22a. The communication unit 23 includes a communication interface capable of communicating with the control apparatus 30, the video processing apparatus 310, and the information processing apparatus 320 based on a predetermined communication standard.

The viewer 211 displays a video based on video data received from the video processing apparatus 310. The head sensors 212 output a signal indicating whether or not the head of the operator is approaching the viewer 211. The microphone 214 accepts voice uttered by the operator and generates audio data corresponding to the voice. The operation panel 204 is a display input unit that performs display of images and acceptance of operations. The limit sensors 231a to 237a output signals indicating whether or not the foot pedals 231 to 237 are stepped on, respectively. The foot sensors 241 to 248 output signals indicating positions of feet relative to the foot pedals.

FIG. 11 is a block diagram showing a functional configuration of the hand controller 220.

The hand controller 220 includes: a servo motor SM1, an encoder EN1, and a servo controller SC1 corresponding to the joint JT1; a servo motor SM2, an encoder EN2, and a servo controller SC2 corresponding to the joint JT2; a servo motor SM3, an encoder EN3, and a servo controller SC3 corresponding to the joint JT3; a servo motor SM4, an encoder EN4, and a servo controller SC4 corresponding to the joint JT4; a servo motor SM5, an encoder EN5, and a servo controller SC5 corresponding to the joint JT5; a servo motor SM6, an encoder EN6, and a servo controller SC6 corresponding to the joint JT6; a servo motor SM7, an encoder EN7, and a servo controller SC7 corresponding to the joint JT7; and a servo motor SM8, a Hall sensor HS, and a servo controller SC8 corresponding to the joint JT8.

The servo motors SM1 to SM8 rotate the joints JT1 to JT8 about corresponding axes, respectively. The encoders EN1 to EN7 output signals corresponding to angles of rotation of the joints JT1 to JT7 about corresponding axes, respectively, and the hall sensor HS outputs a signal corresponding to an opening angle of the pair of movable plates 228. Signals from the encoders EN1 to EN7 are output to the controller 21 via the servo controllers SC1 to SC7, and a signal from the Hall sensor HS is output to the controller 21 via the servo controller SC8. The servo controllers SC1 to SC8 may drive the servo motors SM1 to SM8 according to control of the controller 21 such that output signals of the encoders EN1 to EN7 and the Hall sensor HS become desired values, respectively.

The encoders EN1 to EN7 may detect angles of rotation of the servo motors SM1 to SM7, respectively, or alternatively may detect angles of rotation of the joints JT1 to JT7 themselves. Further, in order to detect the opening angle of the pair of movable plates 228, other sensors may optionally be used instead of or in addition to the hall sensor HS.

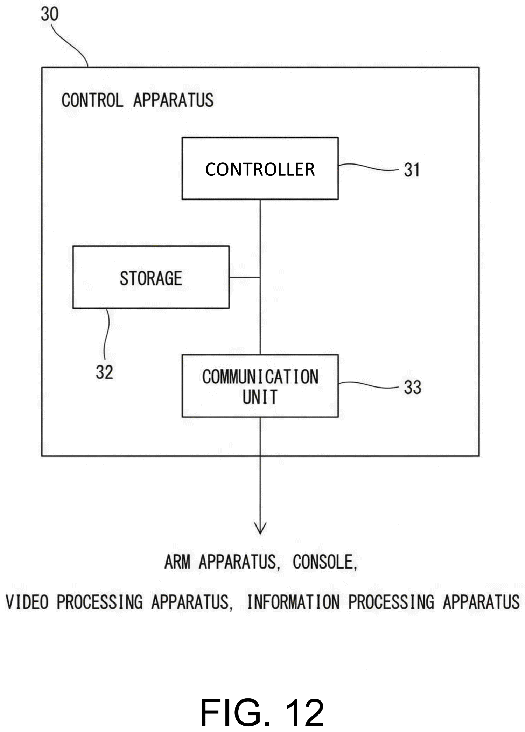

FIG. 12 is a block diagram showing a functional configuration of a control apparatus 30.

The control apparatus 30 includes a controller 31, a storage 32, and a communication unit 33. The controller 31 is composed of, for example, a CPU or an FPGA. The controller 31 controls each part of the hardware of the control apparatus 30 and executes various processes by executing computer programs stored in the storage 32. The storage 32 is composed of, for example, an HDD or an SSD. The communication unit 33 includes a communication interface capable of communicating with the arm apparatus 10, the console 20, the video processing apparatus 310, and the information processing apparatus 320 based on a predetermined communication standard.

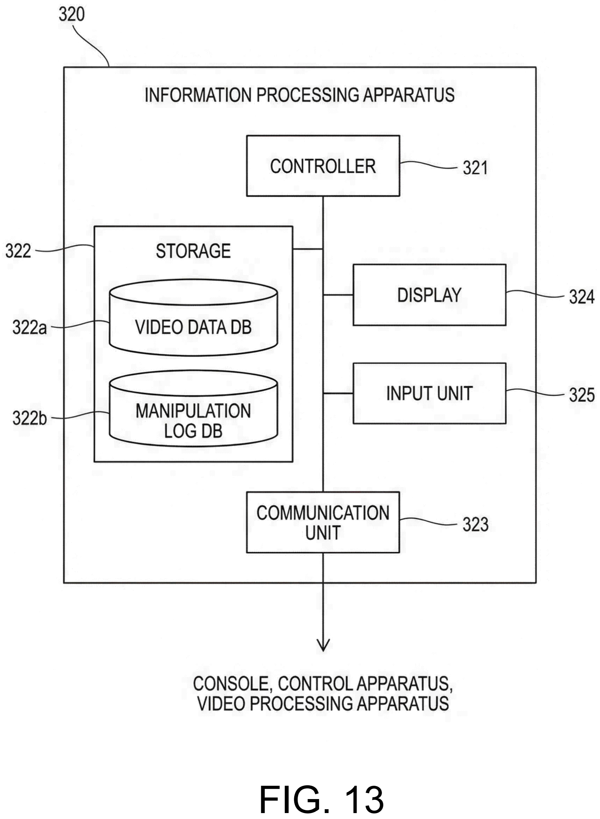

FIG. 13 is a block diagram showing a functional configuration of an information processing apparatus 320.

The information processing apparatus 320 includes a controller 321, a storage 322, a communication unit 323, a display 324, and an input unit 325. The controller 321 is composed of, for example, a CPU. The controller 321 controls each part of the hardware of the information processing apparatus 320 and executes various processes by executing computer programs stored in the storage 322. The storage 322 is composed of, for example, an HDD or an SSD. A video data DB (video data database) 322a and a manipulation log DB (manipulation log database) 322b are stored in the storage 322.

Video data obtained by the endoscope 122 during a surgical procedure using the surgical assistance robot 100 and a manipulation log of the hand controller 220 of the console 20 during the surgical procedure are stored in the video data DB 322a and the manipulation log DB 322b, respectively. The communication unit 323 includes a communication interface capable of communicating with the console 20, the control apparatus 30, and the video processing apparatus 310 based on a predetermined communication standard (for example, Ethernet standard).

During a surgical procedure using the surgical assistance robot 100, the controller 21 of the console 20 accepts a manipulation of the console 20 and transmits manipulation information corresponding to the accepted manipulation to the control apparatus 30. The control apparatus 30 drives the arm apparatus 10 based on the manipulation information received from the console 20. Further, video data related to an image captured by the endoscope 122 during the surgical procedure is transmitted from the video processing apparatus 310 to the console 20.

The control apparatus 30 transmits the manipulation information received from the console 20 to the information processing apparatus 320 as a manipulation log, and the video processing apparatus 310 transmits the video data obtained by the endoscope 122 to the information processing apparatus 320. The manipulation log and video data transmitted to the information processing apparatus 320 are stored in the storage 322 of the information processing apparatus 320 and are used for duplication of the manipulations by a surgeon during a surgical procedure.

Next, information transmitted during the surgical procedure using the surgical assistance robot 100 will be described. Here, "during the surgical procedure" refers to a state in which the surgical instruments and an endoscope are attached to the arms 111 to 114 and the console 20 is capable of operating the surgical instruments and the endoscope. For example, it may refer to a period from when sensors 141 to 144 detect that surgical instruments and an endoscope are attached to the arms 111 to 114 until when the sensors 141 to 144 detect that a surgical instrument or the endoscope is removed from the arms 111 to 114.

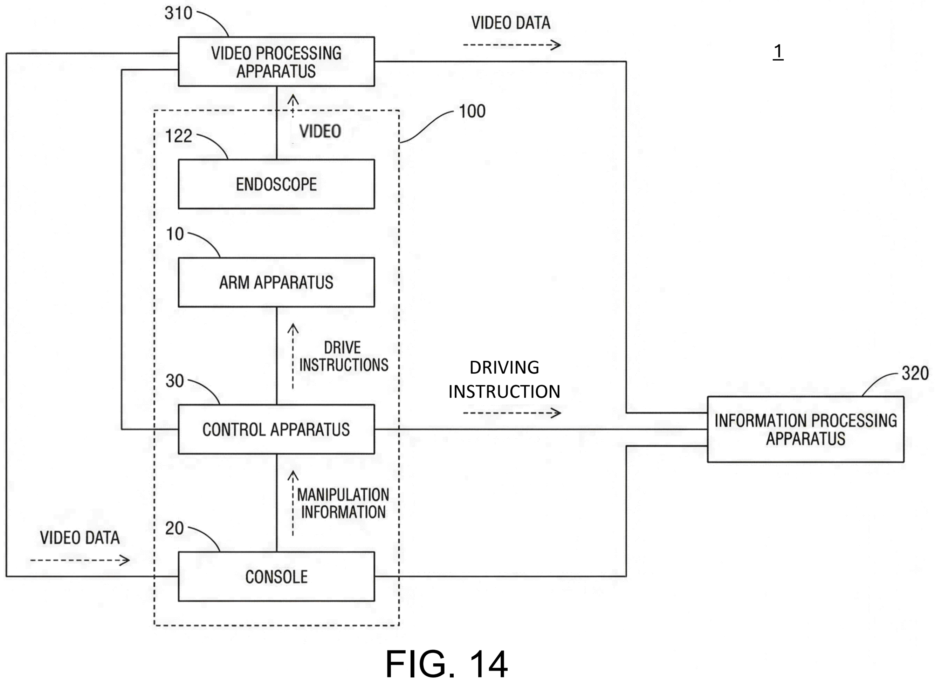

FIG. 14 is a diagram showing the configuration of the surgical system 1 shown in FIG. 2, with information transmitted during a surgical procedure superimposed thereon.

During a surgical procedure by the surgical assistance robot 100, the console 20 displays a video based on video data received from the video processing apparatus 310 on the viewer 211, and transmits manipulation information performed on the console 20 by an operator such as a doctor to the control apparatus 30. The control apparatus 30 transmits a driving instruction to the arm apparatus 10 based on the manipulation information received from the console 20. Thereby, the arm apparatus 10 is driven and the surgical procedure is performed. Further, the control apparatus 30 transmits the manipulation information received from the console 20 to the information processing apparatus 320 as the manipulation log.

The video processing apparatus 310 generates video data based on images captured by the endoscope 122. The video processing apparatus 310 may generate the video data by superimposing a graphic showing a state of manipulation of the console 20, for example, a graphic showing a state of manipulation of the foot unit 230, on a video captured by the endoscope 122. The video processing apparatus 310 transmits the generated video data to the console 20 and the information processing apparatus 320. The information processing apparatus 320 stores the video data received from the video processing apparatus 310 and the manipulation log received from the control apparatus 30 in the video data DB 322a and the manipulation log DB 322b, respectively.

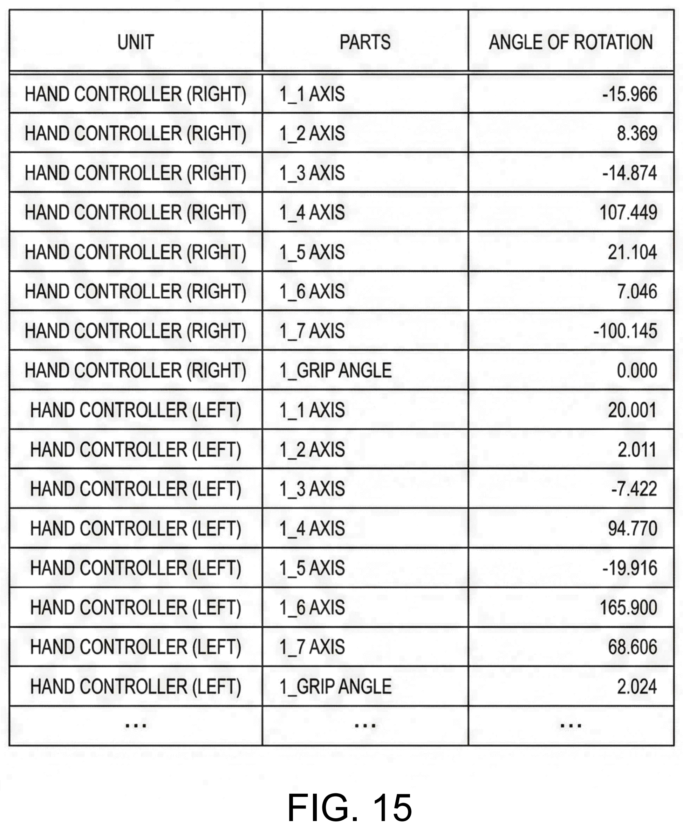

FIG. 15 is a diagram showing an example of manipulation information transmitted from the console 20 to the control apparatus 30.

The controller 21 of the console 20 acquires manipulation information as shown in FIG. 15 at predetermined time intervals. In the manipulation information shown in this example, the information reflecting the angle of rotation is numerically recorded for each of the joints in association with unit information identifying either of the hand controllers 220 of the console 20 and part information identifying one of the joints (JT1 to JT8) in the hand controller 220. The information reflecting the angle of rotation of each joint is acquired based on signals output from sensors (encoders EN1 to EN7, Hall sensor HS) corresponding to each joint. The information reflecting the angle of rotation of each joint may be, for example, a difference between a signal output from each sensor (encoder EN1 to EN7, Hall sensor HS) at a previous acquisition timing and a signal output from each sensor (encoder EN1 to EN7, Hall sensor HS) at a current acquisition timing. Alternatively, the information may be a signal output from each sensor (encoder EN1 to EN7, Hall sensor HS) at the current acquisition timing.

As shown in FIG. 15, "1_1 Axis" to "1_7 Axis" of "Hand Controller (Right)" corresponding to the axes A1 to A7 of the joints JT1 to JT7 of the right hand controller 220 are associated with information reflecting angles of rotation of the respective joints JT1 to JT7 obtained from output signals of the encoders EN1 to EN7 of the right hand controller 220. "1_Grip Angle" of "Hand Controller (Right)" corresponding to the axis A8 of the joint JT8 of the right hand controller 220 is associated with information reflecting an angle of rotation of the joint JT8 obtained from an output signal of the Hall sensor HS of the right hand controller 220. Similarly, "1_1 Axis" to "1_7 Axis" of "Hand Controller (Left)" corresponding to the axes A1 to A7 of the joints JT1 to JT7 of the left hand controller 220 are associated with information reflecting angles of rotation of the respective joints JT1 to JT7 obtained from output signals of the encoders EN1 to EN7 of the left hand controller 220. "1_Grip Angle" of "Hand Controller (Left)" corresponding to the axis A8 of the joint JT8 of the left hand controller 220 is associated with information reflecting an angle of rotation of the joint JT8 obtained from an output signal of the Hall sensor HS of the left hand controller 220.

In addition, the controller 21 may acquire, as manipulation information, information obtained from output signals of the limit sensors 231a to 237a corresponding to the foot pedals 231 to 237, information obtained from output signals of the foot sensors 241 to 248, and information on a positioning state of a head of the operator obtained from an output signal of the head sensors 212.

The controller 21 assigns a current date and time managed in the console 20 to manipulation information acquired at predetermined time intervals, and transmits the manipulation information to the control apparatus 30. The predetermined time interval is, for example, 8 milliseconds. The predetermined time interval is not limited to 8 milliseconds and may optionally be set as appropriate.

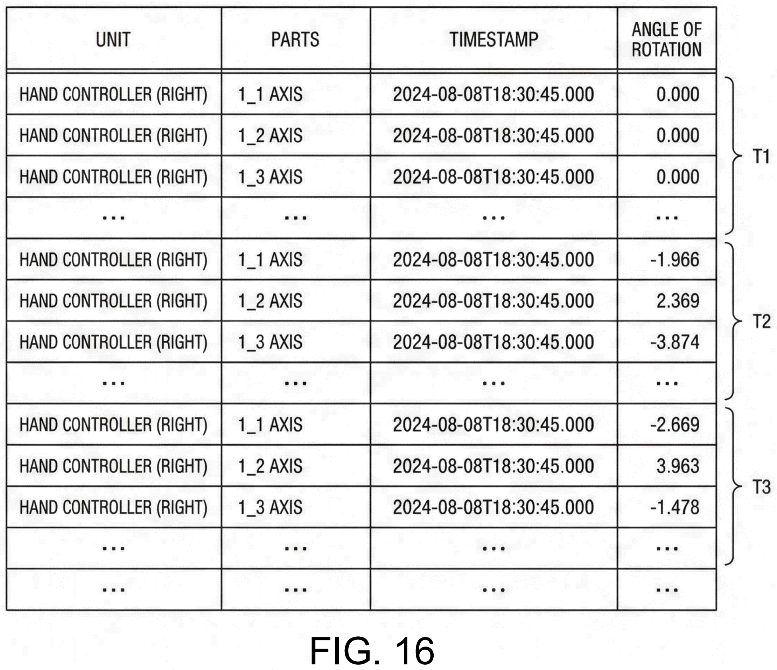

FIG. 16 is a diagram showing manipulation information continuously transmitted from the console 20 to the control apparatus 30 at predetermined time intervals. In the example shown in FIG. 16, manipulation information is acquired in the console 20 at consecutive timings T1, T2, and T3 at 8-millisecond intervals and is transmitted to the control apparatus 30 together with timestamps indicating the current date and time. The manipulation information transmitted to the control apparatus 30 is transmitted as a manipulation log from the control apparatus 30 to the information processing apparatus 320 and stored in the manipulation log DB 322b of the information processing apparatus 320.

The video processing apparatus 310 generates video data based on a video captured by the endoscope 122. A frame rate of the video is set to, for example, 60 frames/second. That is, a video per second is constituted by 60 frames of still images. The video processing apparatus 310 adds a timestamp indicating the current date and time managed in the video processing apparatus 310 as meta-information to each frame, and sequentially transmits the frames to the information processing apparatus 320. The frame rate of the video is not limited to 60 frames/second and may optionally be set as appropriate.

Video data transmitted from the video processing apparatus 310 to the information processing apparatus 320 is stored in the video data DB 322a of the information processing apparatus 320 as a plurality of video files divided in chronological order. The controller 321 of the information processing apparatus 320 may generate one video file at, for example, 10-second intervals and store the same in the video data DB 322a.

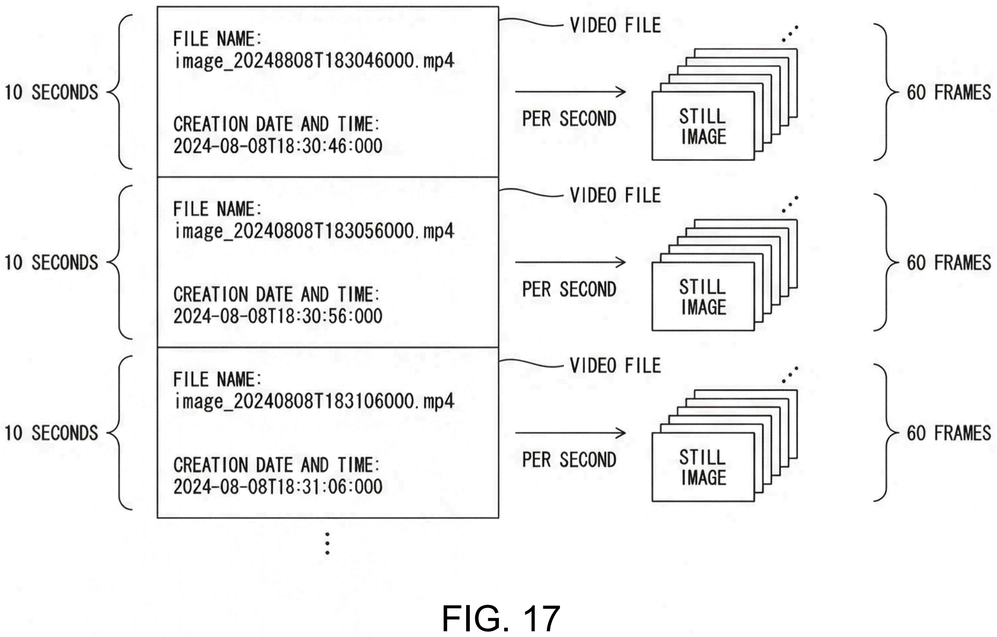

FIG. 17 is a diagram showing a configuration of a plurality of video files stored in the video data DB 322a. As shown in FIG. 17, a date and time indicated by a timestamp added to a head frame included in a video file are associated with the video file stored in the video data DB 322a as a creation date and time of the video file, and a file name of the video file based on the creation date and time of the video file is assigned.

It is preferable that the controller 21 of the console 20 and the video processing apparatus 310 synchronize times of their respective built in clocks with each other. For example, each of the console 20 and the video processing apparatus 310 may be connected to an external NTP server, and the times of the console 20 and the video processing apparatus 310 may be synchronized based on a time acquired from the NTP server. Accordingly, when the manipulation log and video are played back on the console 20, the same scene during the surgical procedure can be synchronized with each other based on the timestamps added to the manipulation log and the video data.

Next, a method for generating information to be transmitted from the information processing apparatus 320 to the console 20 for playback of the manipulation log and video on the console 20 will be described.

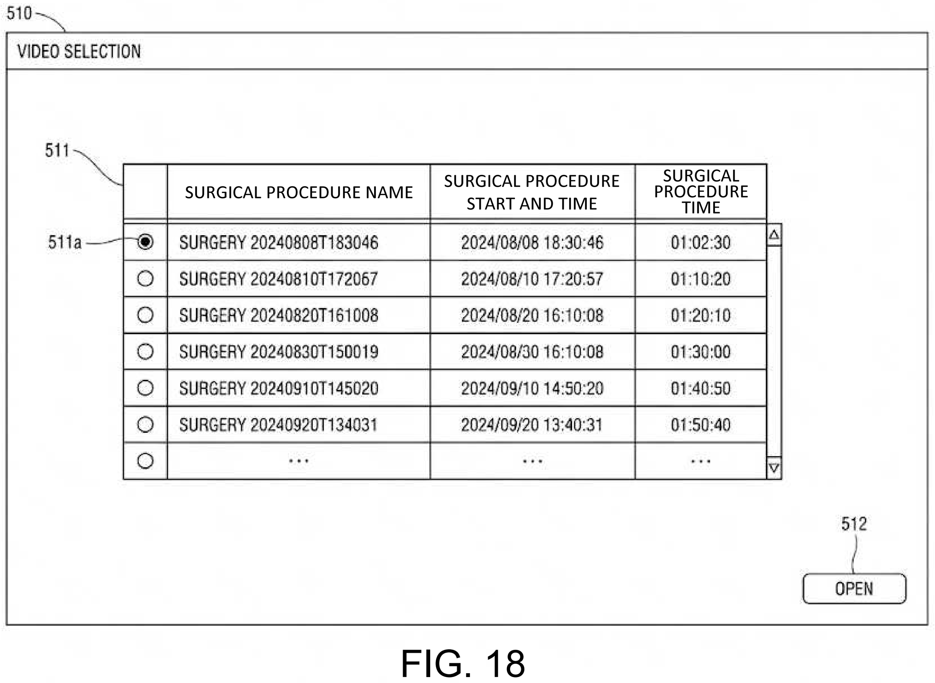

FIG. 18 is a diagram showing a configuration of a video selection screen 510 displayed on the display 324 of the information processing apparatus 320.

The video selection screen 510 includes a video list area 511 and an open button 512. When a user of the information processing apparatus 320 inputs an instruction for displaying the video selection screen 510 via the input unit 325, the controller 321 of the information processing apparatus 320 reads video data of a selected surgical procedure from the video data DB 322a. Based on the creation dates and times of video files, the controller 321 recognizes the video files as a plurality of consecutive video files along a time series as video data related to one surgical procedure, generates a surgical procedure name and a surgical procedure start date and time based on the creation date and time of the first video file of the one surgical procedure, and calculates a surgical procedure time from the total length of the plurality of video files included in the one surgical procedure. Then, the controller 321 generates video list information including the surgical procedure name, the surgical procedure start date and time, and the surgical procedure time for each surgical procedure, and displays the generated video list information in the video list area 511.

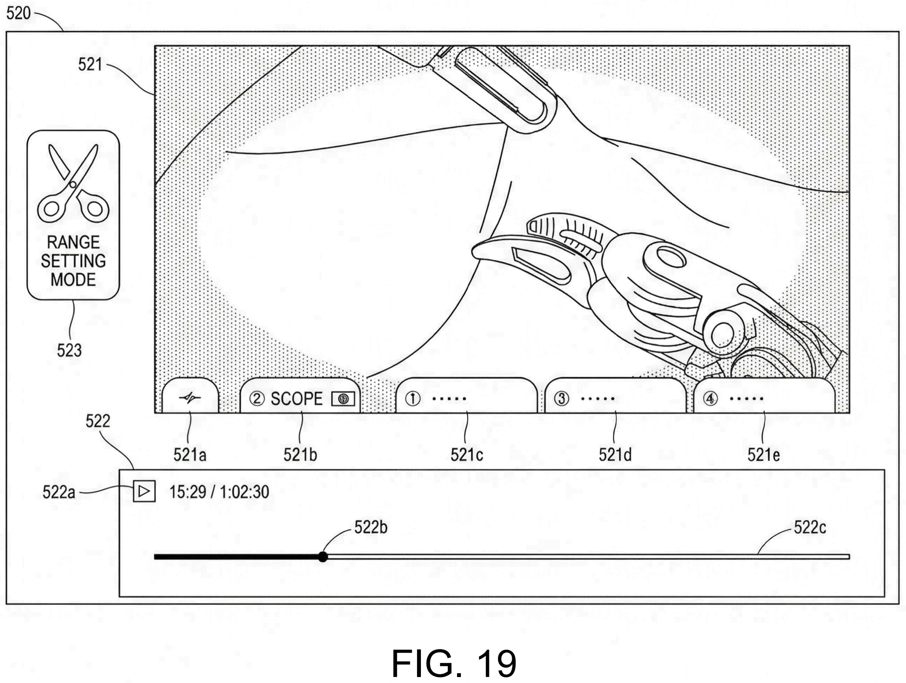

Radio buttons 511a each corresponding to a surgical procedure are provided in the video list area 511. Using the input unit 325, the user selects the radio button 511a for the surgical procedure to be edited to a selected state and selects the open button 512. Thereby, the controller 321 of the information processing apparatus 320 reads video data (a plurality of video files) corresponding to one selected surgical procedure from the video data DB 322a, and displays an edit screen 520 shown in FIG. 19 on the display 324 based on the read video data.

FIG. 19 is a diagram showing a configuration of the edit screen 520 displayed on the display 324 of the information processing apparatus 320.

The edit screen 520 includes an endoscopic video 521, a video control area 522, and a range setting mode button 523.

The endoscopic video 521 is a video based on video data corresponding to one surgical procedure. In a case where the video data corresponding to one surgical procedure includes a plurality of consecutive video files, the endoscopic video 521 is displayed based on the plurality of video files.

The endoscopic video 521 shows a surgical site of a patient undergoing a surgical procedure and surgical instruments operating for the surgical procedure at the surgical site. As shown in FIG. 19, the endoscopic video 521 may include display areas 521a to 521e showing information on surgical instruments and an endoscope attached to the arms 111 to 114 of the arm apparatus 10.

The display area 521a shows a state of manipulation of the foot pedal 232 which is a clutch pedal. When a foot is positioned relative to the foot pedal 232, the outer periphery of the display area 521a is colored. When the foot pedal 232 is stepped on, a color is applied inside the display area 521a. The display area 521b shows a state of manipulation of the foot pedal 233 for manipulating an arm equipped with the endoscope 122. When a foot is positioned relative to the foot pedal 233, a color is applied to an outer periphery of the display area 521b, and when the foot pedal 233 is stepped on, a color is applied inside the display area 521b.

The display area 521c shows states of manipulation of the foot pedals 234 and 235 for manipulating forceps (electric scalpel). When a foot is positioned relative to the foot pedal 234 or 235, the outer periphery of the display area 521c is colored; when the foot pedal 234 is stepped on, a first color is applied inside the display area 521c; and when the foot pedal 235 is stepped on, a second color is applied inside the display area 521c. The first color is, for example, light blue, and the second color is, for example, yellow.

The display area 521d shows states of manipulation of the foot pedals 236 and 237 for manipulating forceps (electric scalpel), and the display area 521e shows states of manipulation of the foot pedals 236 and 237 for manipulating other forceps (electric scalpel). Each time the foot pedal 231 is stepped on, a target of the foot pedals 236 and 237 is switched between the forceps 123 and 124. When a foot is positioned relative to the foot pedal 236 or 237, the outer periphery of a target display area among the display areas 521d and 521e is colored; when the foot pedal 236 is stepped on, a first color is applied inside the target display area among the display areas 521d and 521e; and when the foot pedal 237 is stepped on, a second color is applied inside the target display area among the display areas 521d and 521e.

Further, in the display areas 521c to 521e, for example, names are displayed as information on the surgical instruments 121, 123, and 124 attached to the arms 111, 113, and 114.

The video control area 522 includes a play button 522a, a playback position mark 522b, and a timeline 522c. When the play button 522a is selected, playback and pause of the endoscopic video 521 are switched. The playback position mark 522b is a mark indicating a playback position of video on the timeline 522c. The playback position can be changed by manipulating the playback position mark 522b.

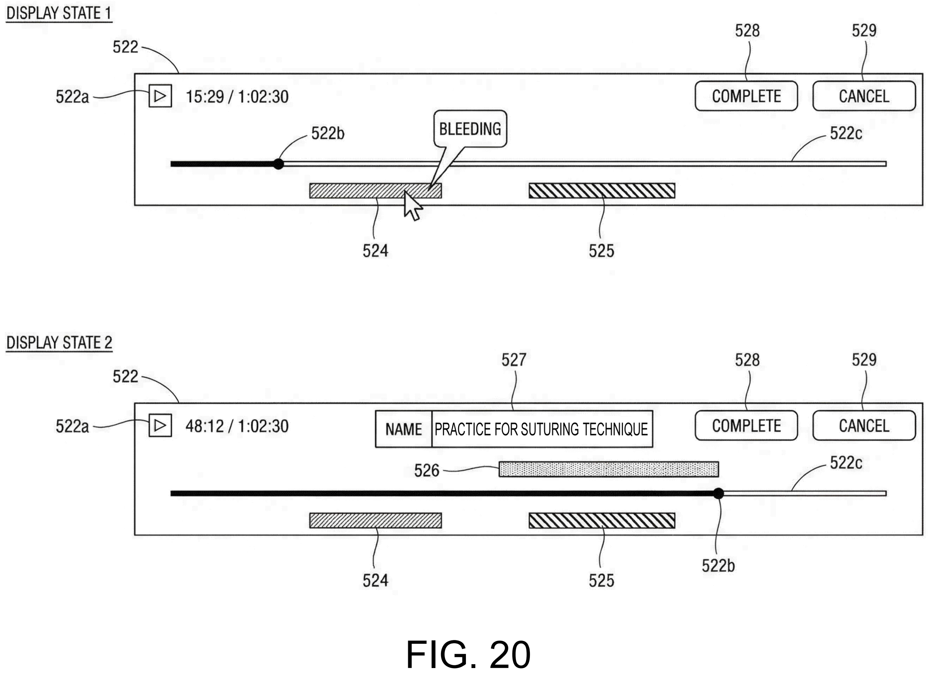

The range setting mode button 523 is a button for switching a display mode of the edit screen 520 to a mode for range setting (trimming) of a video. This mode is used for setting a playback target period for playing back a manipulation log and a video on the console 20. When the range setting mode button 523 is selected by the user, the video control area 522 is displayed as shown in FIG. 20.

The upper and lower parts of FIG. 20 are diagrams showing display states 1 and 2 of a video control area 522, respectively.

When the range setting mode button 523 shown in FIG. 19 is selected, a controller 321 of the information processing apparatus 320 analyzes video data corresponding to the endoscopic video 521 and determines whether or not there is a frame satisfying a predetermined condition. The predetermined condition is a condition for detecting a specific event occurring during a surgical procedure, and is preset in the information processing apparatus 320. Specific events include, for example, a state change such as bleeding at a surgical site and a specific technique such as suturing performed by a surgeon.

For example, the controller 321 refers to a still image of a head frame of the video data every second, recognizes a blood area based on color information of each pixel of the referred still image of the head frame, and quantifies the area. For example, a ratio of the blood area portion is calculated. If an area corresponding to blood of a predetermined ratio or more exists, it is determined that a bleeding event has occurred in this head frame. Similarly, the controller 321 determines that a suturing event has occurred in the head frame if an area corresponding to a needle exists in the referred still image of the head frame. Alternatively, the controller 321 may recognize suturing scene when a dedicated forceps (e.g., needle holder) holding a suturing needle is attached to an arm. The controller 321 may label the images corresponding to a time point at which one or more needle holders are attached to the arm as a suturing scene.

As shown in the upper part of FIG. 20, the controller 321 automatically sets markers along a timeline 522c according to ranges in which predetermined events occur. For example, a marker 524 is a range of the video in which a bleeding event occurs, and a marker 525 is a range of the video in which a suturing event occurs. When a user performs a predetermined operation (for example, an operation of hovering a mouse pointer) on the markers 524 and 525, an event name of the target marker is displayed as a pop-up. In the example of the upper part of FIG. 20, a character string "Bleeding" is displayed in a pop-up for the marker 524.

Further, as shown in the lower part of FIG. 20, the user can manually set a range of the video for a playback target period. The user can set a marker 526 by playing back the endoscopic video 521 to review contents of the surgical procedure and designating a start point and an end point by performing a predetermined operation (for example, right-clicking) on the timeline 522c. In the present embodiment, the start point and the end point can be set in increments of one second. When the marker 526 is set manually, an input area 527 for inputting a name for the marker 526 is displayed in the video control area 522. The user inputs texts such as "Practice for suturing technique" into the input area 527 via the input unit 325.

After setting the markers 524 to 526 and inputting to the input area 527 as shown in FIG. 20, when a complete button 528 is selected by the user, the controller 321 stores, in a storage 322, playback information indicating playback target periods corresponding to these markers. The controller 321 may optionally include a setting date and time of the playback target period in the playback information as a data creation date and time. When the markers 524 and 525 are automatically set, the controller 321 may alternatively (or also) include character strings of "bleeding" and "suturing" in the playback information as playback data names, respectively. When the marker 526 is set, the controller 321 may also include the character string of the input area 527 in the playback information as a playback data name. In addition to ranges of these markers and character strings, information indicating whether the markers are set automatically or manually may also be included in the playback information. When a cancel button 529 is selected by the operator, these markers are discarded, and an edit screen 520 is returned to the state shown in FIG. 19.

When setting playback target periods corresponding to the automatically set markers 524 and 525 as ranges of video, the controller 321 may optionally set the playback target periods by adding a predetermined period to at least one of before and after the ranges shown by the markers 524 and 525. For example, when setting a range of video in which a bleeding event occurs, the controller 321 may automatically set the playback target period by setting the playback start position to a predetermined period (such as 5 seconds) is added before the bleeding scene as a playback start position and an end point of the bleeding scene as a playback end position, so that the operator can also review manipulations of the hand controller 220 that caused the bleeding. Further, the controller 321 may automatically set the playback target period with a position where a predetermined period (such as 5 seconds) is added after the bleeding scene as the playback end position, so that the operator can review treatment manipulations after bleeding.

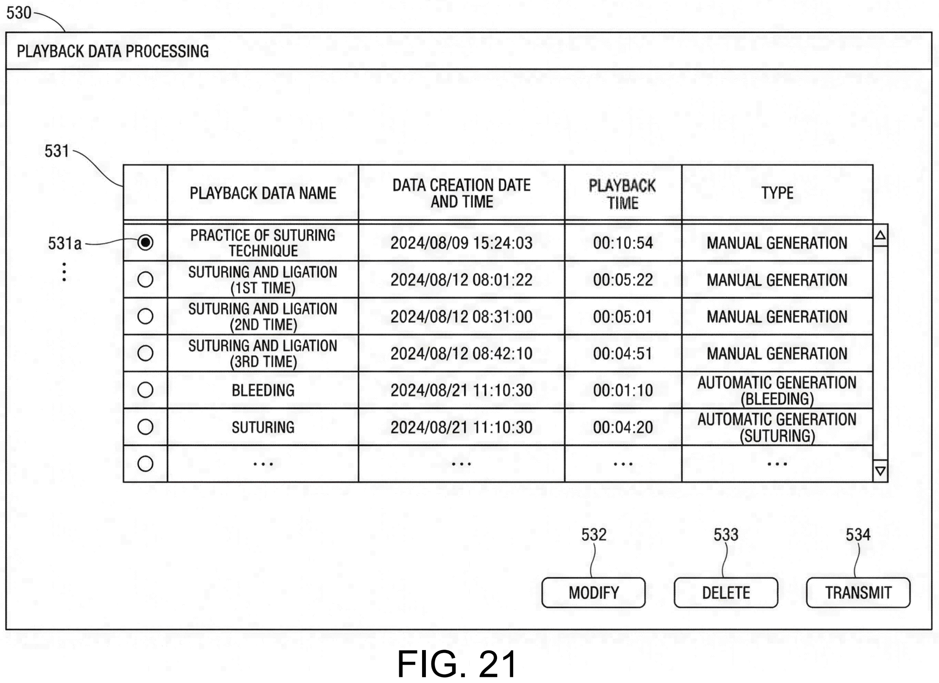

FIG. 21 is a diagram showing a configuration of a playback data processing screen 530 displayed on a display 324 of the information processing apparatus 320.

The playback data processing screen 530 includes a playback data list area 531, a modify button 532, a delete button 533, and a transmit button 534.

When a user of the information processing apparatus 320 inputs an instruction to display the playback data processing screen 530 via the input unit 325, the controller 321 of the information processing apparatus 320 reads all playback information stored in the storage 322 and displays the read playback information in the playback data list area 531.

Radio buttons 531a each corresponding to playback information are provided in the playback data list area 531. The user of the information processing apparatus 320 selects the radio button 531a of the playback information to be processed using the input unit 325 and presses the modify button 532, the delete button 533, or the transmit button 534.

When the modify button 532 is selected, the controller 321 displays the edit screen 520 shown in FIG. 19 again on the display 324. When the delete button 533 is selected, the controller 321 deletes only target playback information from the storage 322 without deleting video data and a manipulation log of the corresponding surgical procedure.

When the transmit button 534 is selected, the controller 321 generates video data and a manipulation log corresponding to a playback target period indicated by the selected playback information as playback data, and transmits the generated playback data to the console 20.

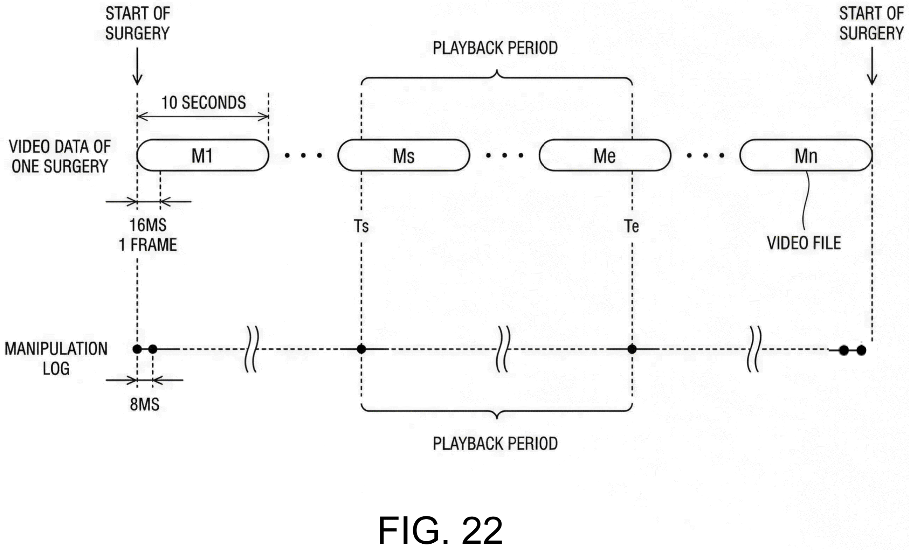

FIG. 22 is a diagram showing generation of video data and a manipulation log corresponding to a playback target period.

As described above, one video file has a length of, for example, 10 seconds, and comprises, for example, 60 frames of still images per second. A timestamp is added to each frame. Further, the manipulation log of the hand controller 220 is acquired at intervals of, for example, 8 milliseconds, and a timestamp is also attached to each manipulation log.

In the example shown in FIG. 22, it is assumed that video data of one surgical procedure includes a plurality of video files M1 to Mn, and a playback target period set in the information processing apparatus 320 is a range from a start timing Ts to an end timing Te. The controller 321 of the information processing apparatus 320 generates video data and a manipulation log corresponding to the playback target period from the start timing Ts to the end timing Te as playback data to be transmitted to the console 20.

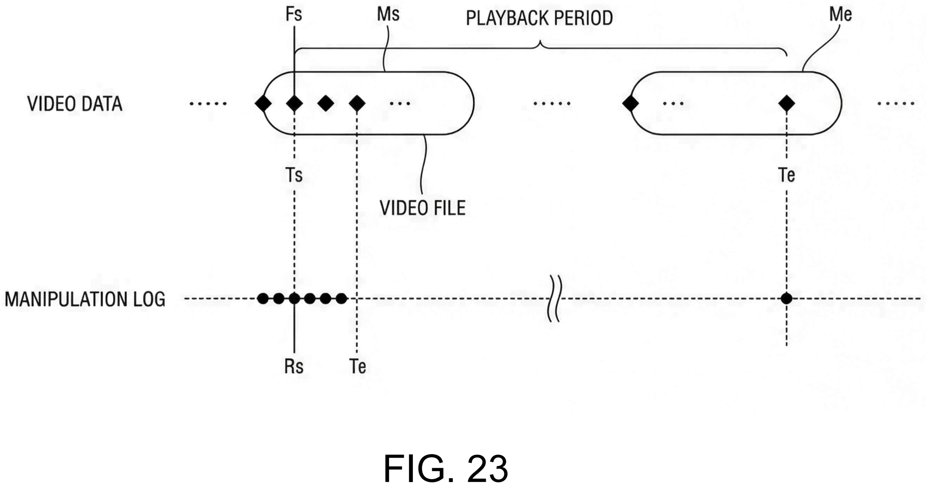

FIG. 23 is a diagram for explaining details of generation of video data and a manipulation log corresponding to the playback target period from the start timing Ts to the end timing Te.

First, the controller 321 reads, from the video data DB 322a, a series of video frames from a video frame to which a timestamp corresponding to a start timing Ts is added to a video frame corresponding to an end timing Te, and to identify the series of video frames as playback video data.

Next, the controller 321 searches for a manipulation log corresponding to the start timing Ts (manipulation log Rs in FIG. 23) and a manipulation log corresponding to the end timing Te from a manipulation log DB 322b.

If there is no manipulation log to which a timestamp matching the start timing Ts is assigned, the controller 321 identifies, for example, a manipulation log to which a timestamp closest in time to the start timing Ts is assigned as the manipulation log corresponding to the start timing Ts. Alternatively, a manipulation log with the closest timestamp before the start timing Ts may be identified, or optionally a manipulation log with the closest timestamp after the start timing Ts may be identified. Further, if there is no manipulation log to which a timestamp matching the end timing Te is assigned, the controller 321 identifies, for example, a manipulation log to which a timestamp closest in time to the end timing Te is assigned as the manipulation log corresponding to the end timing Te. Alternatively, a manipulation log with the closest timestamp before the end timing Te may be identified, or optionally a manipulation log with the closest timestamp after the end timing Te may be identified.

Thereby, the controller 321 reads a series of manipulation logs from the manipulation log corresponding to the start timing Ts to the manipulation log corresponding to the end timing Te from the manipulation log DB 322b, and identifies them as the manipulation log for playback.

The controller 321 transmits the playback data (video data and manipulation log) corresponding to the identified playback target period to the console 20. In this way, by transmitting playback data corresponding to a partial period rather than data of the entire surgical procedure to the console 20, only portions that an operator such as a trainee needs to pay attention to can be efficiently played back on the console 20.

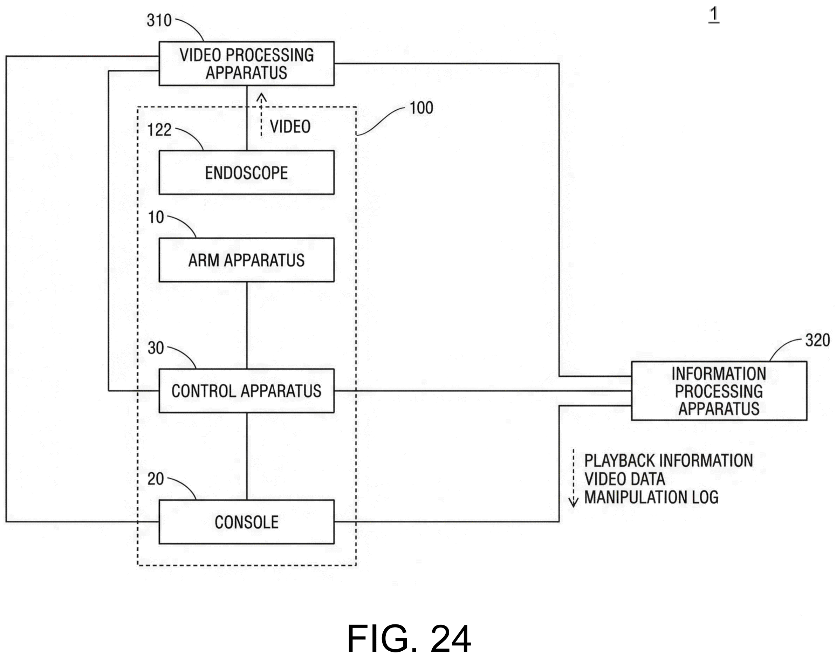

FIG. 24 is a diagram showing the configuration of the surgical system 1 shown in FIG. 2, with information transmitted from the information processing apparatus 320 to the console 20 superimposed thereon.

As shown in FIG. 24, the information processing apparatus 320 transmits playback data (video data and manipulation log) and playback information to the console 20. The playback information includes a name of the playback data and the like. When receiving the playback data (video data and manipulation log) and playback information from the information processing apparatus 320, the console 20 stores the received information in a playback data DB 22a.

When duplicating the manipulations of the console by the surgeon during the surgical procedure, the controller 21 of the console 20 reads playback data (video data and manipulation log) and playback information from the playback data DB 22a, and drives each joint of the hand controller 220 according to the read manipulation log while playing back the read video on a viewer 211. At this time, the arm apparatus 10 and the video processing apparatus 310 are not driven.

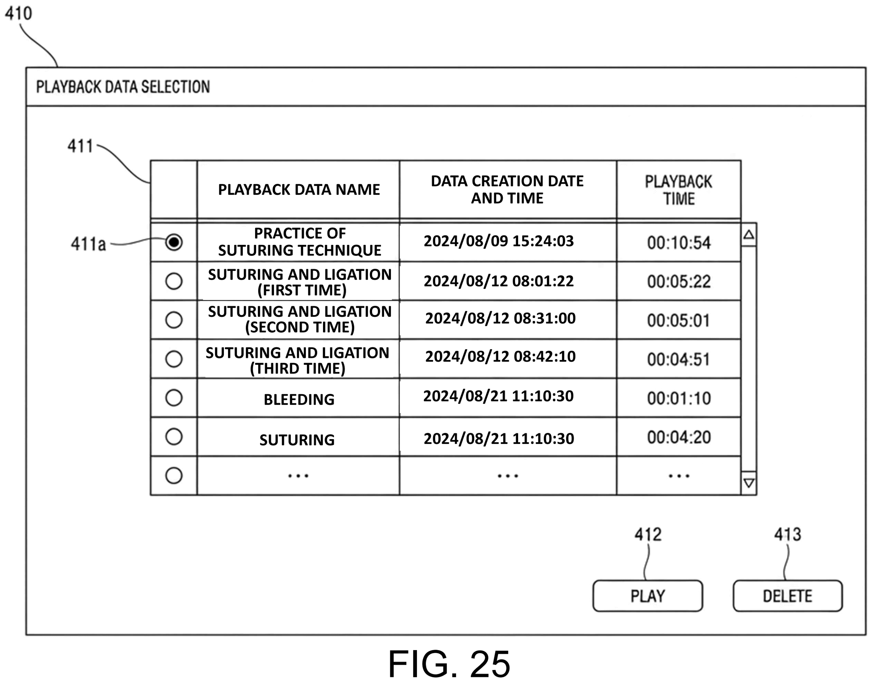

FIG. 25 is a diagram showing a configuration of a playback data selection screen 410 displayed on an operation panel 204 of the console 20 when playing back playback data (video data and manipulation log).

The playback data selection screen 410 includes a playback data list area 411, a play button 412, and a delete button 413.

When an operator such as a trainee inputs an instruction to display the playback data selection screen 410 via the operation panel 204, the controller 21 of the console 20 reads all playback information stored in the playback data DB 22a of a storage 22 and displays the read playback information in the playback data list area 411.

Radio buttons 411a each corresponding to playback information are provided in the playback data list area 411. The trainee sets the radio button 411a to a selected state using the operation panel 204 and selects the play button 412 or the delete button 413.

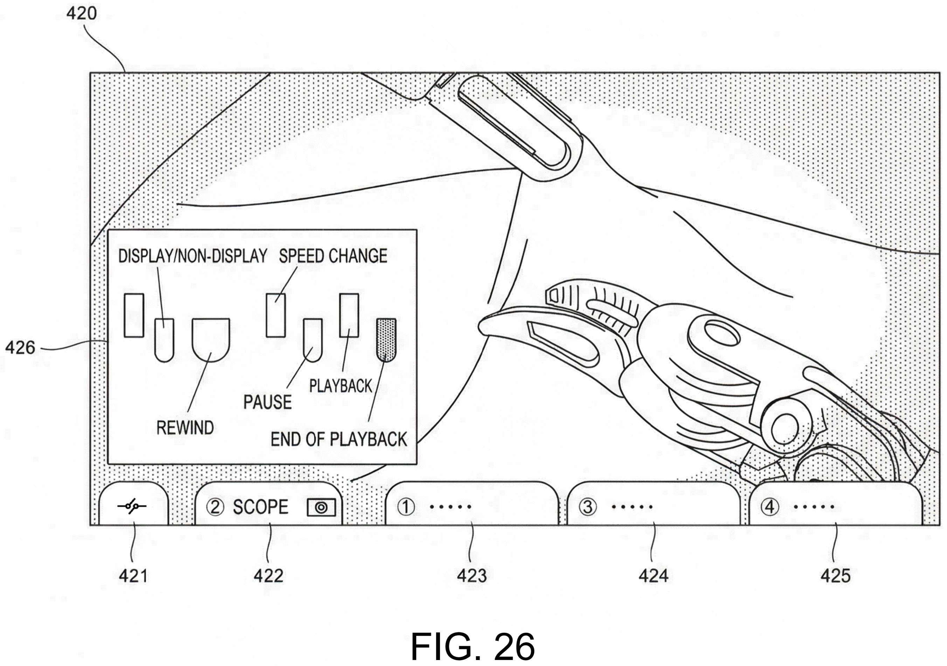

In response to an instruction to start playback of the playback data, the controller 21 reads the playback data (video data and manipulation log) corresponding to the selected playback information from the playback data DB 22a, plays back and displays a video based on the read video data on the viewer 211 as an endoscopic video 420 as shown in FIG. 26, and drives each joint of the hand controllers 220 for the left and right hand based on the read manipulation log. When the delete button 413 is selected on the playback data selection screen 410, the controller 21 may optionally delete the selected playback information and the playback data (video data and manipulation log) corresponding to the playback information from the playback data DB 22a.

The controller 21 may accept an operation of the play button 412 as an instruction to start playback of playback data and start playback of the playback data according to the operation of the play button 412, or may start playback of the playback data after a predetermined time (for example, 5 seconds) has elapsed since the play button 412 was operated. Further, in order to prevent playback from starting in a state where an operator such as a trainee is not sitting at the console 20, the controller 21 may start playback of the playback data on condition that the play button 412 is selected and the head sensors 212 detect that the operator is sitting at the console 20. Further, so that playback can start with the operator sitting at the console 20 in a posture for the surgical procedure, the controller 21 may start playback of the playback data on condition that the play button 412 is selected and the limit sensors 231a to 237a detect that the operator has operated any of the foot pedals 231 to 237 of the console 20. Further, the operation of the play button 412 may not be essential for starting playback of playback data, and playback of the playback data may start on condition that the head sensors 212 detect that the operator is sitting at the console 20 and the limit sensors 231a to 237a detect that the operator has operated any of the foot pedals 231 to 237 of the console 20.

In response to the instruction to start playback of the playback data (video data and manipulation log), the controller 21 controls servo motors SM1 to SM8 to move the hand controller 220 to an initial position. Subsequently, the controller 21 plays back the video by sequentially displaying frames included in the video data for playback on the viewer 211 from the frame corresponding to the start timing Ts, in accordance with a frame rate of the video data. Further, the controller 21 drives each joint of the hand controller 220 by sequentially supplying the manipulation log for playback to the servo motors SM1 to SM8 of the hand controller 220 from the manipulation log corresponding to the start timing Ts, in accordance with an acquisition interval of the manipulation log.

When the end timing Te is reached, the controller 21 stops playing back the video and driving each joint of the hand controller 220. Thereby, the controller 21 performs driving based on the video data and the manipulation log synchronously in a range from the start timing Ts to the end timing Te.

FIG. 26 is a diagram showing a configuration of the endoscopic video 420 played back and displayed on the viewer 211 of the console 20.

Similar to the endoscopic video 521 shown in FIG. 19, the endoscopic video 420 may include display areas 421 to 425 showing information on surgical instruments and an endoscope attached to the arms 111 to 114 of the arm apparatus 10.

A guidance area 426 showing operations performed by stepping on a foot pedal may be displayed on the endoscopic video 420. When the second foot pedal 232 from the left among seven foot pedals arranged horizontally is stepped on, the controller 21 may switch between display and non-display of the guidance area 426. When the third to seventh foot pedals 233 to 237 from the left are stepped on, the controller 21 may rewind, change speed, pause, play, and end playback of the endoscopic video 420, respectively. In response to the foot pedal 233 corresponding to rewinding being stepped on, the controller 21 rewinds the video. According to the number of times the foot pedal 234 corresponding to speed change is stepped on, the controller 21 may change the playback speed of the video stepwise to, for example, 0.5x speed, 0.75x speed, 1x speed, 1.25x speed, 1.5x speed, or the like. In response to the foot pedal 235 corresponding to pause being stepped on, the controller 21 stops playing back the video and correspondingly stops driving each joint of the hand controller 220. After pausing, in response to the foot pedal 236 corresponding to playback being stepped on, the controller 21 resumes playing back the video and correspondingly resumes driving each joint of the hand controller 220.

The controller 21 may stop driving each joint of the hand controller 220 during rewinding of the video. After rewinding of the video, the controller 21 may resume playback of the playback data (video data and manipulation log) in response to the foot pedal 236 corresponding to playback being stepped on. At that time, after the controller 21 controls the servo motors SM1 to SM8 to move the hand controllers 220 to the initial position, the controller 21 may control the viewer 211 to resume playback of the video, and resume driving of joints of the hand controllers 220. The controller 21 identifies a video frame and a manipulation log to which a timestamp corresponding to a resumption timing is assigned, sequentially plays back the video on the viewer 211 from the identified video frame, and sequentially supplies the manipulation log to the servo motors SM1 to SM8 from the identified manipulation log, thereby resuming driving of each joint of the hand controller 220. If there is no manipulation log to which a timestamp matching the resumption timing is assigned, a manipulation log with a timestamp temporally closest to the resumption timing may be identified as the manipulation log corresponding to the resumption timing.

The controller 21 may accept, as operations for playback of the endoscopic video 420, not only rewinding, changing speed, pausing, playing, and ending playback as operations for playback of the endoscopic video 420, and may optionally accept, for example, a skip operation for skipping forward the endoscopic video 420. That is, the controller 21 may accept a skip operation for skipping a specific portion during playback of the video and jumping to a next scene or a specified time. When accepting the skip operation, the controller 21 may control the servo motors SM1 to SM8 to move the hand controller 220 to the initial position before resuming playback of the playback data (video data and manipulation log), and then resume playback of the video and driving of each joint of the hand controller 220.

Even when changing the playback speed of the video, the controller 21 may synchronize the frames included in the video data with driving of each joint of the hand controller 220. Specifically, playback of the video is realized by adjusting frame intervals according to a specified speed. For example, if the speed is 1.5x, the frame interval is multiplied by 2/3 relative to the added timestamp. Driving of the hand controller 220 is realized by adjusting a speed at which the manipulation log is supplied to the servo motors SM1 to SM8 according to the specified speed. For example, if the speed is 1.5x, each supply timing interval of the manipulation log is multiplied by 2/3. Accordingly, playback of the video and driving of the hand controller 220 can be synchronized with each other. Thereby, an operator such as a trainee can efficiently review manipulations of the surgeon during the surgical procedure by changing the playback speed of the video according to contents of the surgical procedure. For example, by making the playback speed faster than usual during a simple surgical procedure and making the playback speed slower than usual during a complex and difficult surgical procedure, confirmation of surgical contents can proceed efficiently.

The controller 21 may stop driving the hand controller 220 when the playback speed of the video is faster than the normal speed. Thereby, unnecessary driving of the hand controller 220 can be prevented while efficiently searching for a surgical scene to be viewed by increasing the playback speed of the video.

By referring to the endoscopic video 420 played back on the viewer 211, an operator such as a trainee can refer to the same video as the video actually referred to by the surgeon during the surgical procedure. Further, by driving each joint of the left and right hand controllers 220 based on the manipulation log corresponding to the playback period of the endoscopic video 420, the operator sitting at the console 20 and placing fingers on the hand controller 220 can physically experience the manipulations of the hand controller 220 actually performed by the surgeon during the surgical procedure through his/her own fingers.

Although the motions of the surgical instruments are reflected in the endoscopic video 420, manipulations of the hand controllers 220 itself are not shown, and therefore it has conventionally been difficult to confirm the manipulations. However, by duplicating the manipulations of the hand controllers 220 as in the present embodiment, an operator such as a trainee can learn a specific manipulation method of the surgeon. For example, while a proficient expert surgeon tends to manipulate the hand controllers 220 for the right and left hands at regular intervals during a surgical procedure, a beginner surgeon often narrows the interval. Further, an expert often manipulates the hand controllers 220 within a range close to his or her body. Thereby, by duplicating the manipulations of the hand controller 220 not reflected in the endoscopic video 420, a beginner can experience the manipulations of the expert and deeply understand the manipulation method in an actual surgical procedure.

Further, the controller 21 also duplicates manipulations of the pair of movable plates (grips) 228 related to opening and closing operations of forceps based on the manipulation log. Therefore, a delicate technique of the surgeon for the forceps during the surgical procedure can be reviewed by duplication of the movement of the pair of movable plates 228.

Further, an operator such as a trainee can also understand manipulations of the foot pedals 232 to 237 by the surgeon during the surgical procedure and what surgical instrument is attached to the arm by referring to the display areas 421 to 425 in the endoscopic video 420.

While driving the hand controller 220 based on the manipulation log, an actual angle of rotation of each joint of the hand controller 220 may differ from an angle of rotation commanded in the manipulation log due to force applied to the hand controller 220 by the operator. When detecting that the actual angle of rotation detected by sensors (encoders EN1 to EN7, Hall sensor HS) corresponding to each joint differs from the angle of rotation instructed in the manipulation log, the controller 21 may optionally (or alternatively) perform correction at a next control cycle according to a difference therebetween and control the servo motors SM1 to SM8 so as to maintain an appropriate angle of rotation.

<Embodiment 2> In Embodiment 1, one console 20 is shared for manipulation during a surgical procedure and playback of playback data, but a console for playing back playback data may be provided separately from a console for performing manipulation during a surgical procedure.

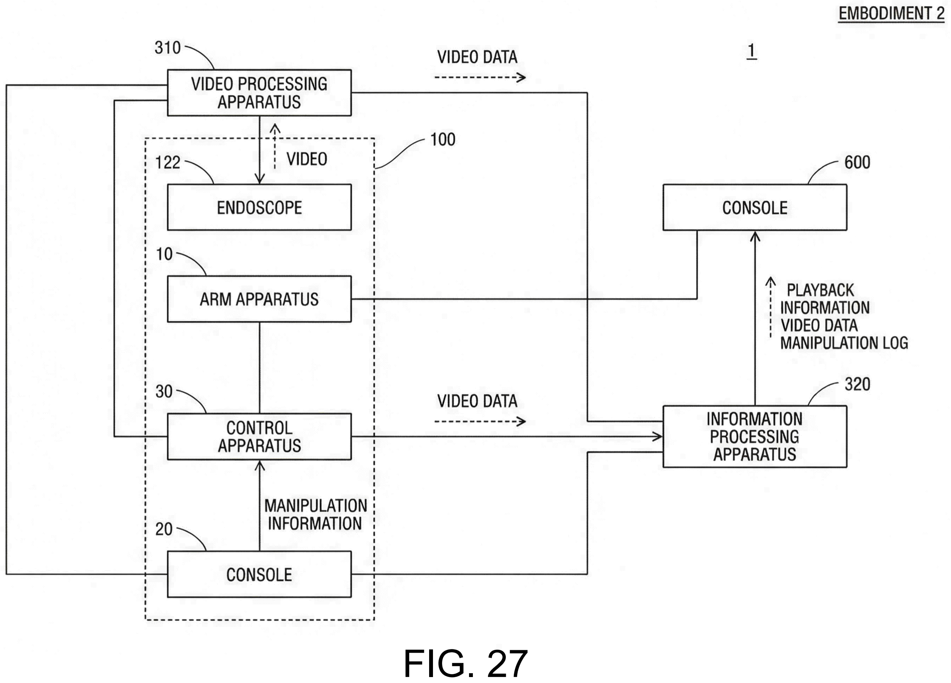

FIG. 27 is a block diagram showing a configuration of a surgical system 1 according to Embodiment 2.

The surgical system 1 of the present embodiment includes a console 600 for playing back playback data, in addition to the configuration of Embodiment 1 shown in FIG. 2. The console 600 has the same configuration as the console 20 of Embodiment 1. The console 600 is communicatively connected to the information processing apparatus 320. Further, the playback data DB 22a stored in the storage 22 of the console 20 in Embodiment 1 is stored in the storage 22 of the console 600.

In the present embodiment, similarly to Embodiment 1, video data transmitted from the video processing apparatus 310 and a manipulation log transmitted from the control apparatus 30 are stored in the video data DB 322a and the manipulation log DB 322b of the information processing apparatus 320. Then, playback data (video data and manipulation log) and playback information are transmitted from the information processing apparatus 320 to the console 600 and stored in the playback data DB 22a of the console 600. In the present embodiment, a video is played back and displayed on the viewer 211 of the console 600, and each joint of the hand controller 220 of the console 600 is driven based on the manipulation log.

In the present embodiment, playback data (video data and manipulation log) may be transmitted not only to the console 600 but also to the console 20. Further, in the present embodiment, by transmitting playback data (video data and manipulation log) only to the console 600, the console 20 may be used exclusively for surgical manipulations and the console 600 may alternatively be used exclusively for playback of playback data.

<Other Modifications>In the above embodiment, the timestamp of the video frame is added in the video processing apparatus 310 and the timestamp of the manipulation log is added in the console 20, but the present invention is not limited thereto. For example, if a video frame transmitted from the video processing apparatus 310 and a manipulation log transmitted from the control apparatus 30 record the same moment during a surgical procedure, and they arrive at the information processing apparatus 320 simultaneously, the information processing apparatus 320 may add timestamps to the manipulation log and the video frame. Accordingly, even in a case where the times of the clocks of the controller 21 of the console 20 and the video processing apparatus 310 are shifted from each other, a common timestamp is added to each of the video frame and the manipulation log in the information processing apparatus 320, and the video data and the manipulation log can be synchronized with each other based on the common timestamp.

In the above embodiment, each of joints of the hand controllers 220 is driven in accordance with the manipulation log synchronously with playback of the video on the viewer 211 of the console 20, but synchronization is not necessarily required. For example, playback of the video on the viewer 211 may precede driving of the hand controller 220 so that an operator such as a trainee can view the video played back on the viewer 211 and then view the manipulations of the hand controller 220 in that scene.

In the above embodiment, video data based on one surgical procedure is divided into a plurality of video files and stored, but is not limited thereto and may be stored as one video file.

In the above embodiment, the date and time indicated by the timestamp of the head frame included in the video file is assigned as the creation date and time of the video file (see FIG. 17), but the date and time indicated by the timestamp of the last frame of the video file may be added as the creation date and time of the video file.

In the above embodiment, in the example shown in FIG. 22, the start timing Ts and the end timing Te are both in the middle of the video data regardless of whether manual or automatic settings are used. However, the present invention is not limited thereto, and the start timing Ts may be a start time point of the video data (the start date and time of the first video file), and the end timing Te may be an end time point of the video data (the end date and time of the last video file).

In Embodiment 2, the console 600 is configured similarly to the console 20, but the console 600 is not necessarily configured in the same manner. The console 600 (second console) only needs to include a viewer 211 that displays a video based on video data stored in the information processing apparatus 320. Further, it is sufficient that operations corresponding to the manipulation log stored in the information processing apparatus 320 can be duplicated by the hand controller 220 of the console 600. That is, the console 600 (second console) only needs to include joints JT1 to JT8 having the same structure as the joints JT1 to JT8 of the hand controllers 220 of the console 20 (first console).

In the above embodiment, operations performed by stepping on the foot pedals 232 to 237 are shown in the guidance area 426 (see FIG. 26), but instead of the guidance display of the guidance area 426, voice corresponding to the guidance display of the guidance area 426 may be output from a speaker 215 provided in the console 20 to notify an operator such as a trainee. Further, although the controller 21 of the console 20 accepts processes such as changing the speed of the video, pausing, and playing through manipulations of the foot pedals 233 to 237, these may be accepted through audio input via the speaker 215.

In the above embodiment, video data, manipulation logs, and playback information are stored in the storage 22 of the console 20 or the console 600, but is not limited thereto and may be stored in an external device (for example, a hard disk, a database server, or the like) directly connected to the console 20 or the console 600. Alternatively, video data, manipulation logs, and playback information stored in the storage 322 of the information processing apparatus 320 may be transmitted to the console 20 or the console 600 on demand. For example, a playback data selection screen 410 as shown in FIG. 25 may be displayed on the display 324 of the information processing apparatus 320, and playback data (video data and manipulation log) selected on the playback data selection screen 410 may be transmitted from the information processing apparatus 320 to the console 20 or the console 600, and the console 20 or the console 600 may play back the received playback data.

In the above embodiment, playback information is transmitted to the consoles 20 and 600 together with video data and manipulation logs. However, the present invention is not limited thereto, and information corresponding to the playback information may be added to meta-information of the video data. In this case, transmission of the playback information becomes unnecessary.

In the above embodiment, the hand controller 220 is driven based on the manipulation log, but is not limited thereto, and the foot pedals 231 to 237 may be driven based on a manipulation log representing manipulation histories of the foot pedals 231 to 237. In this case, each of the foot pedals 231 to 237 may be provided with a driving mechanism for driving the foot pedal in a pushing-down direction or a pushing-up direction. As such a driving mechanism, a pneumatic or electric actuator may be used.

The embodiments of the present invention can be appropriately modified in various ways within the scope of the technical ideas shown in the claims.

### Remarks

The present disclosure includes following items 1-19.

Item 1. A system for a surgical assistance robot configured to perform a surgical procedure on a patient using a surgical instrument attached to an arm, the system comprising:
a console comprising (i) a hand controller configured to be manipulated by a surgeon to remotely control an operation of the surgical instrument, wherein the hand controller comprises a plurality of joints rotatable by a manipulation of the hand controller, and (ii) a viewer configured to display images captured by an endoscope, the images showing a surgical site of the patient and the operation of the surgical instrument;
a storage configured to store (a) a video generated from the images which are captured during the surgical procedure and (b) a manipulation log representing a series of the manipulations of the hand controller during the surgical procedure, wherein the manipulation log comprises an angle of rotation of each of the joints according to the manipulation; and
a transmitter configured to transmit, to the console, the video and the manipulation log stored in the storage,
wherein the console is configured to rotate the joints according to the manipulation log in association with a playback of the video on the viewer for duplication of the manipulations by the surgeon during the surgical procedure.

Item 2. The system according to claim 1, wherein the console is configured to start rotating the joints based on the manipulation log in response to starting playback of the video on the viewer.

Item 3. The system according to claim 1, wherein the console is configured to stop rotating the joints in response to stopping playback of the video on the viewer.

Item 4. The system according to claim 1, wherein the console further includes an input unit configured to control the playback of the video, and wherein the console is configured to control rotating the joints in accordance with the playback of the video controlled via the input unit.

Item 5. The system according to claim 4, wherein the console includes a foot pedal, and wherein the foot pedal is used as the input unit.

Item 6. The system according to claim 1, wherein the console is configured to synchronize the rotation of the joints with the playback of the video.

Item 7. The system according to claim 1, wherein the manipulation log includes a timestamp associated with the information related to the angle of rotation, and wherein the console is configured to identify the timestamp corresponding to the start time of the playback of the video and to start rotating the joints based on the manipulation log that includes the identified timestamp.

Item 8. The system according to claim 1, wherein the console is configured to rotate the joints in accordance with speed of the playback of the video.

Item 9. The system according to claim 1, wherein the storage is configured to store the video indicating the surgical site undergoing the surgical procedure and a motion of the surgical instrument at the surgical site.

Item 10. The system according to claim 1, further comprising a processor configured to extract, based on the video and the manipulation log stored in the storage, a portion of the video corresponding to a playback period and the manipulation log corresponding to the playback period, wherein the transmitter is configured to transmit the video and the manipulation log generated by the processor to the console.

Item 11. The system according to claim 10, wherein the processor is configured to receive a designation of the playback period, and configured to extract the portion of the video corresponding to the designated playback period.

Item 12. The system according to claim 10, wherein the processor is configured to generate the portion of the video corresponding to the playback period to include a frame satisfying a predetermined condition among a plurality of frames constituting the video captured by the endoscope during the surgical procedure.

Item 13. The system according to claim 12, wherein the condition is the condition for detecting at least one of a change at the surgical site and a specific technique performed by the surgeon during the surgical procedure.

Item 14. The system according to claim 1, wherein the hand controller includes a first hand controller for a right hand and a second hand controller for a left hand.

Item 15. The system according to claim 1, wherein the transmitter is configured to transmit the video including first video and second video, and transmit the manipulation log including a first manipulation log corresponding to the first video and a second manipulation log corresponding to the second video, and wherein the console is configured to receive a selection of either the first video or the second video, and configured to rotate the joints in accordance with the manipulation log while playing the designated video on the viewer.

Item 16. The system according to claim 1, wherein the surgical instrument includes forceps including an openable and closable tip, wherein the hand controller includes a grip configured to open and close the tip of the forceps, wherein the manipulation log includes information reflecting an opening angle of the grip, and wherein the console is configured to rotate the grip in accordance with the manipulation log corresponding to the playback period of the video.

Item 17. The system according to claim 1, wherein the console is a first console used for the surgical procedure.

Item 18. The system according to claim 1, wherein:
the console includes a first console used for the surgical procedure and a second console, wherein, the first console comprises a first viewer and a first hand controller having a plurality of first joints; and
the second console comprises a second viewer and a second hand controller having a plurality of second joints,
the storage is configured to store the video and the manipulation log representing a series of the manipulations of the first hand controller during the surgical procedure;
the transmitter is configured to transmit the video and the manipulation log to the second console; and the second console is configured to rotate the second joints according to the manipulation log in association with a playback of the video on the second viewer for duplication of the manipulations of the first hand controller by the surgeon.

Item 19. A method for controlling a console for a surgical assistance robot, the console including a hand controller configured to operate a surgical instrument attached to an arm of the surgical assistance robot and a viewer configured to display images showing a surgical site of a patient and an operation of the surgical instrument, the method comprising:
storing (a) a video generated from the images which are captured during a surgical procedure, and (b) a manipulation log representing a series of manipulations by a surgeon of the hand controller during the surgical procedure, wherein the manipulation log comprises an angle of rotation of each of a plurality of joints of the hand controller; and
rotating the joints of the hand controller according to the manipulation log in association with a playback of the video on the viewer for duplication of the manipulations by the surgeon during the surgical procedure.

## Claims

1. A system for a surgical assistance robot configured to perform a surgical procedure on a patient using a surgical instrument attached to an arm, the system comprising:
a console comprising (i) a hand controller configured to be manipulated by a surgeon to remotely control an operation of the surgical instrument, wherein the hand controller comprises a plurality of joints rotatable by a manipulation of the hand controller, and (ii) a viewer configured to display images captured by an endoscope, the images showing a surgical site of the patient and the operation of the surgical instrument;
a storage configured to store (a) a video generated from the images which are captured during the surgical procedure and (b) a manipulation log representing a series of the manipulations of the hand controller during the surgical procedure, wherein the manipulation log comprises an angle of rotation of each of the joints according to the manipulation; and
a transmitter configured to transmit, to the console, the video and the manipulation log stored in the storage,
wherein the console is configured to rotate the joints according to the manipulation log in association with a playback of the video on the viewer for duplication of the manipulations by the surgeon during the surgical procedure.

2. The system according to claim 1, wherein the console is configured to start rotating the joints based on the manipulation log in response to starting playback of the video on the viewer.

3. The system according to claim 1, wherein the console is configured to stop rotating the joints in response to stopping playback of the video on the viewer.

4. The system according to claim 1, wherein the console further includes an input unit configured to control the playback of the video, and wherein the console is configured to control rotating the joints in accordance with the playback of the video controlled via the input unit.

5. The system according to claim 4, wherein the console includes a foot pedal, and wherein the foot pedal is used as the input unit.

6. The system according to claim 1, wherein the console is configured to synchronize the rotation of the joints with the playback of the video.

7. The system according to claim 1, wherein the manipulation log includes a timestamp associated with the information related to the angle of rotation, and wherein the console is configured to identify the timestamp corresponding to the start time of the playback of the video and to start rotating the joints based on the manipulation log that includes the identified timestamp.

8. The system according to claim 1, wherein the console is configured to rotate the joints in accordance with speed of the playback of the video.

9. The system according to claim 1, wherein the storage is configured to store the video indicating the surgical site undergoing the surgical procedure and a motion of the surgical instrument at the surgical site.

10. The system according to claim 1, further comprising a processor configured to extract, based on the video and the manipulation log stored in the storage, a portion of the video corresponding to a playback period and the manipulation log corresponding to the playback period, wherein the transmitter is configured to transmit the video and the manipulation log generated by the processor to the console.

11. The system according to claim 10, wherein the processor is configured to receive a designation of the playback period, and configured to extract the portion of the video corresponding to the designated playback period.

12. The system according to claim 10, wherein the processor is configured to generate the portion of the video corresponding to the playback period to include a frame satisfying a predetermined condition among a plurality of frames constituting the video captured by the endoscope during the surgical procedure.

13. The system according to claim 12, wherein the condition is the condition for detecting at least one of a change at the surgical site and a specific technique performed by the surgeon during the surgical procedure.

14. The system according to claim 1, wherein the hand controller includes a first hand controller for a right hand and a second hand controller for a left hand.

15. The system according to claim 1, wherein the transmitter is configured to transmit the video including first video and second video, and transmit the manipulation log including a first manipulation log corresponding to the first video and a second manipulation log corresponding to the second video, and wherein the console is configured to receive a selection of either the first video or the second video, and configured to rotate the joints in accordance with the manipulation log while playing the designated video on the viewer.

16. The system according to claim 1, wherein the surgical instrument includes forceps including an openable and closable tip, wherein the hand controller includes a grip configured to open and close the tip of the forceps, wherein the manipulation log includes information reflecting an opening angle of the grip, and wherein the console is configured to rotate the grip in accordance with the manipulation log corresponding to the playback period of the video.

17. The system according to claim 1, wherein the console is a first console used for the surgical procedure.

18. The system according to claim 1, wherein:
the console includes a first console used for the surgical procedure and a second console, wherein,
the first console comprises a first viewer and a first hand controller having a plurality of first joints; and
the second console comprises a second viewer and a second hand controller having a plurality of second joints,
the storage is configured to store the video and the manipulation log representing a series of the manipulations of the first hand controller during the surgical procedure;
the transmitter is configured to transmit the video and the manipulation log to the second console; and
the second console is configured to rotate the second joints according to the manipulation log in association with a playback of the video on the second viewer for duplication of the manipulations of the first hand controller by the surgeon.

19. A method for controlling a console for a surgical assistance robot, the console including a hand controller configured to operate a surgical instrument attached to an arm of the surgical assistance robot and a viewer configured to display images showing a surgical site of a patient and an operation of the surgical instrument, the method comprising:
storing (a) a video generated from the images which are captured during a surgical procedure, and (b) a manipulation log representing a series of manipulations by a surgeon of the hand controller during the surgical procedure, wherein the manipulation log comprises an angle of rotation of each of a plurality of joints of the hand controller; and
rotating the joints of the hand controller according to the manipulation log in association with a playback of the video on the viewer for duplication of the manipulations by the surgeon during the surgical procedure.
